# EUROPÄISCHE PATENTSCHRIFT

(11) **EP 2 371 848 B1**
(45) Veröffentlichungstag und Bekanntmachung des Hinweises auf die Patenterteilung: **18.04.2018**
(21) Anmeldenummer: 11002013.8
(22) Anmeldetag: 18.05.2005
(51) Int. Cl.: C07K 14/47, C07K 16/30, A61K 39/395, A61P 35/00

(54) **Differentiell in Tumoren exprimierte Genprodukte und deren Verwendung**
Differential in tumour gene products and use of same
Produits géniques d'expression différentielle dans les tumeurs et leur utilisation

(30) Priorität: 18.05.2004 DE 102004024617
(43) Veröffentlichungstag der Anmeldung: 05.10.2011
(62) Teilanmeldung aus: 05745559.4
(73) Patentinhaber: Ganymed Pharmaceuticals AG, 55131 Mainz (DE)
(72) Erfinder: Türeci, Özlem, 55116 Mainz (DE); Sahin, Ugur, 55116 Mainz (DE); Koslowski, Michael, 65933 Frankfurt (DE); Fritz, Stefan, 55237 Flonheim (DE); Geppert, Harald-Gerhard, 42697 Solingen (DE)
(74) Vertreter: Schnappauf, Georg

(56) Entgegenhaltungen:
- WO-A-00/15659
- WO-A-02/18576
- WO-A2-2004/047863
- NIIMI T ET AL: "CLAUDIN-18, A NOVEL DOWSTREAM TARGET GENE FOR THE T/EBP/NKX2.1 HOMEODO", MOLECULAR AND CELLULAR BIOLOGY, AMERICAN SOCIETY FOR MICROBIOLOGY, WASHINGTON, US, Bd. 21, Nr. 21, 1. Januar 2001 (2001-01-01), Seiten 7380-7390, XP002375751, ISSN: 0270-7306, DOI: 10.1128/MCB.21.21.7380-7390.2001

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen. An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = Histokompatibilitätsantigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektonnechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster oder zirkulierende Autoantikörper, wurden genutzt, um die respektiven Antigene zu klonieren.

Durch diese Ansätze sind in den letzten Jahren eine Vielzahl von Antigenen in verschiedenen Neoplasien definiert worden. Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumor-assoziiert exprimierter Antigene und der dafür kodierenden Nukleinsäuren verfolgt. Diese Strategie beruht auf der Tatsache, dass bestimmte Gene, die Organ-spezifisch, z.B. ausschließlich im Kolon-, Lungen- oder Nieren-Gewebe, exprimiert werden, in den entsprechenden Organen auch von Tumorzellen und darüber hinaus in anderen Geweben in Tumorzellen ektop und unerlaubt reaktiviert werden. Durch Datamining wird zunächst eine möglichst komplette Liste aller bekannten Organ-spezifischen Gene aufgestellt und diese sodann durch Expressionsanalysen mittels spezifischer RT-PCR auf ihre aberrante Aktivierung in unterschiedlichen Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumorspezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Organ-spezifischer Gene zu nutzen und diese sodann auf Expression in Tumoren zu evaluieren.

Somit betrifft die vorliegende Lehre in einem Aspekt eine Strategie zur Identifizierung von Gewebe-spezifischen und differentiell in Tumoren exprimierten Genen. Diese Strategie kombiniert Datamining von öffentlichen Sequenzbanken ("*in silico*") mit darauf folgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Tumor-Gene. Diese sind bisher als rein Organ-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser Tumor-assoziierten Gene und der dadurch kodierten Genprodukte erfolgte unabhängig von einer immunogenen Wirkung.

Die identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: 1-8, 41-44, 51-59, 84, 117, 119 und 138, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein identifiziertes Tumor-assoziiertes Antigen eine Aminosäuresequenz auf, die von einer Nukleinsäure kodiert wird, die aus der Gruppe bestehend aus SEQ ID NO: 1-8, 41-44, 51-59, 84, 117, 119 und 138 ausgewählt ist. In einer weiteren bevorzugten Ausführungsform umfasst ein identifiziertes Tumor-assoziiertes Antigen eine Aminosäuresequenz, die aus der Gruppe bestehend aus SEQ ID NO: 9-19, 45-48, 60-66, 85, 90-97, 100-102, 105, 106, 111-116, 118, 120, 123, 124, 135-137, 139 und 142-150, einem Teil oder Derivat davon ausgewählt ist.

Die vorliegende Offenbarung betrifft allgemein die Verwendung von offenbarungsgemäß identifizierten Tumor-assoziierten Antigenen oder von Teilen oder Derivaten davon, von dafür kodierenden Nukleinsäuren oder von Nukleinsäuren, die gegen die kodierenden Nukleinsäuren gerichtet sind, oder von Antikörpern, die gegen die identifizierten Tumor-assoziierten Antigene oder Teile oder Derivate davon gerichtet sind, für die Therapie und Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der offenbarungsgemäß identifizierten Tumor-assoziierten Antigene vorliegt.

Die Offenbarung betrifft auch Nukleinsäuren und Genprodukte, die tumorzellassoziiert exprimiert werden.

Desweiteren betrifft die vorligende technische Lehre Genprodukte, d.h. Nukleinsäuren und Proteine bzw. Peptide, die durch verändertes Spleißen (Spleißvarianten) bekannter Gene bzw. durch veränderte Translation unter Nutzung alternativer offener Leserahmen entstehen. In diesem Aspekt werden Nukleinsäuren offenbart, die eine Nukleinsäuresequenz ausgewählt aus der Gruppe bestehend aus den Sequenzen gemäß SEQ ID NO: 3-5 des Sequenzprotokolls umfassen. Außerdem betrifft die offenbarte Lehre in diesem Aspekt Proteine bzw. Peptide, die eine Aminosäuresequenz ausgewählt aus der Gruppe bestehend aus den Sequenzen gemäß SEQ ID NO: 10 und 12-14 des Sequenzprotokolls umfassen. Die offenbarten Spleißvarianten sind als Targets für die Diagnostik und Therapie von Tumorerkrankungen verwendbar.

Insbesondere betrifft die vorliegende Lehre die Aminosäuresequenz gemäß SEQ ID NO: 10 des Sequenzprotokolls, die durch einen erfindungsgemäß identifizierten alternativen offenen Leseraster kodiert wird und sich von der vorbeschriebenen Protein-Sequenz (SEQ ID NO: 9) durch 85 zusätzliche Aminosäuren am N-Terminus des Proteins unterscheidet.

Für die Entstehung von Spleißvarianten können verschiedenste Mechanismen ursächlich sein, beispielsweise
- die Nutzung variabler Transkriptionsinitiationsstellen,
- die Nutzung zusätzlicher Exons,
- das vollständige oder unvollständige Ausspleißen von einzelnen oder mehreren Exons,
- durch Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei Tumor-assoziierten Spleißvarianten können Tumor-assoziierte Transkripte und Tumor-assoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvarianten-spezifischen Oligonukleotiden erfolgen. Als Oligonukleotide eignen sich insbesondere Paare von Primern, von denen mindestens einer unter stringenten Bedingungen an die Region der Spleißvariante bindet, die Tumor-assoziiert ist. Offenbarungsgemäß geeignet sind die in den Beispielen für diesen Zweck beschriebenen Oligonukleotide, insbesondere Oligonukleotide, die eine Sequenz ausgewählt aus SEQ ID NO: 34-36, 39, 40 und 107-110 des Sequenzprotokolls aufweisen bzw. umfassen. Zum Nachweis eignen sich alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. In diesem Aspekt betrifft die offenbarte Lehre insbesondere Peptide, die eine Sequenz ausgewählt aus SEQ ID NO: 17-19, 111-115, 120 und 137 des Sequenzprotokolls aufweisen bzw. umfassen und dagegen gerichtete spezifische Antikörper. Die Detektion von Tumorzellen kann auch durch Antikörper erfolgen, die tumorspezifisch veränderte Glykosylierungsvarianten erkennen. Für die Generierung von derartigen Antikörpern können Peptidregionen genutzt werden, die sich in Tumorzellen und gesunden Zellen glykosylierungsbedingt unterscheiden. In diesem Aspekt betrifft die Offenbarung insbesondere Peptide, die eine Sequenz ausgewählt aus SEQ ID NO: 17-19, 111-115, 120, 137 und 142-145, des Sequenzprotokolls aufweisen bzw. umfassen und dagegen gerichtete spezifische Antikörper. Durch endogene Deglykosylierung von N-gekoppelten Zuckerresten wird Asparagin in Asparaginsäure transformiert. Erfindungsgemäß können daher die hier beschriebenen Proteine tumorspezifisch sequenzverändert sein und damit andere biochemische und Antikörper-Bindungseigenschaften aufweisen. In diesem Aspekt betrifft die offenbarte Lehre insbesondere Peptide, die eine Sequenz ausgewählt aus SEQ ID NO: 146-150 des Sequenzprotokolls aufweisen bzw. umfassen und dagegen gerichtete spezifische Antikörper. Für die Immunisierung eignen sich besonders die Aminosäuren, die deutliche Epitopunterschiede zu der (den) Variante(n) des Genprodukts aufweisen, welche(s) bevorzugt in gesunden Zellen gebildet wird (werden). Der Nachweis der Tumorzellen mit Antikörpern kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben (vgl. z.B. Kessler JH, et al. 2001, Sahin et al., 1997) und basieren ebenfalls auf der Nutzung von Oligo- oder Polypeptiden, die die Spleißvarianten-spezifischen Epitope beinhalten oder Nukleinsäuren, die für diese kodieren. Oligo- oder Polypeptiden, die die Spleißvarianten-spezifischen Epitope beinhalten, oder Nukleinsäuren, die für diese Polypeptide kodieren, sind auch als pharmazeutisch wirksame Substanzen bei der aktiven Immuntherapie (Vakzinierung, Vakzintherapie) verwendbar.

Vorliegend werden auch Proteine beschrieben, die sich durch Art und Menge ihrer sekundären Modifikationen in Normal- und Tumorgewebe unterscheiden (z.B. Durand & Seta, 2000; Clin. Chem. 46: 795-805; Hakomori, 1996; Cancer Res. 56: 5309-18).

Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western-Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

Erfindungsgemäß konnte für Claudin-18 eine differentielle Glykosylierung in Tumoren nachgewiesen werden. Gastrointestinale Karzinome, Pankreaskarzinome, Ösophagustumoren, Prostatatumoren als auch Lungentumoren weisen eine weniger glykosylierte Form von Claudin-18 auf. Die Glykosylierung in gesunden Geweben maskiert Proteinepitope von Claudin-18, die auf Tumorzellen aufgrund fehlender Glykosylierung freigelegt sind. Entsprechend lassen sich erfindungsgemäß Liganden und Antikörper selektieren, die an diese Domänen binden. Derartige Liganden und Antikörper binden erfindungsgemäß nicht an das Claudin-18 auf gesunden Zellen, da hier die Epitope durch die Glykosylierung verdeckt sind.

Ähnlich wie oben für von Tumor-assoziierten Spleißvarianten abgeleitete Proteinepitope beschrieben kann somit die differenzielle Glycosylierung zur Unterscheidung von Normal- und Tumorzellen mit diagnostischer wie auch therapeutischer Intention genutzt werden.

In einem Aspekt betrifft die Offenbarung eine pharmazeutische Zusammensetzung umfassend ein Mittel, das das offenbarungsgemäß identifizierte Tumor-assoziierte Antigen erkennt und vorzugsweise selektiv für Zellen ist, die eine Expression oder abnormale Expression eines offenbarungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter oder Toxin-konjugierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein offenbarungsgemäß identifiziertes Tumor-assoziiertes Antigen ist. Die Erkennung muss nicht direkt mit einer Hemmung von Aktivität oder Expression des Antigens einhergehen. In diesem Aspekt der offenbarten technischen Lehre dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zellen lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die offenbarte Lehre eine pharmazeutische Zusammensetzung umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein offenbarungsgemäß identifiziertes Tumor-assoziiertes Antigen ist.

Des weiteren betrifft die offenbarte Lehre eine pharmazeutische Zusammensetzung, die ein Mittel umfasst, das bei einer Verabreichung selektiv die Menge an Komplexen zwischen einem HLA-Molekül und einem Peptidepitop aus dem offenbarungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein offenbarungsgemäß identifiziertes Tumor-assoziiertes Antigen ist.

Des weiteren betrifft die offenbarte Lehre eine pharmazeutische Zusammensetzung, die einen oder mehrer Bestandteile umfasst, die aus der Gruppe ausgewählt sind, bestehend aus (i) einem offenbarungsgemäß identifizierten Tumor-assoziierten Antigen oder einem Teil davon, (ii) einer Nukleinsäure, die für ein offenbarungsgemäß identifiziertes Tumor-assoziiertes Antigen oder einen Teil davon kodiert, (iii) einem Antikörper, der an ein offenbarungsgemäß identifiziertes Tumor-assoziiertes Antigen oder einen Teil davon bindet, (iv) einer Antisense-Nukleinsäure, die spezifisch mit einer Nukleinsäure, die für ein offenbarungsgemäß identifiziertes Tumor-assoziiertes Antigen kodiert, hybridisiert, (v) einer Wirtszelle, die ein offenbarungsgemäß identifiziertes Tumor-assoziiertes Antigen oder einen Teil davon exprimiert, und (vi) isolierten Komplexen zwischen einem offenbarungsgemäß identifizierten Tumor-assoziierten Antigen oder einem Teil davon und einem HLA-Molekül.

Eine Nukleinsäure, die für ein offenbarungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die alle durch kombinatorische Techniken hergestellt werden können. Der Antikörper kann mit einem therapeutisch oder diagnostisch nützlichen Mittel oder Stoff gekoppelt sein.

Eine in einer pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das offenbarungsgemäß identifizierte Tumor-assoziierte Antigen kodiert, umfassen.

In weiteren Ausführungsformen bindet ein durch eine offenbarungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Cytokin oder einem Chemokin ausgewählt sein. Eine pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren betrifft die offenbarte Lehre Verfahren zur Behandlung, Diagnose und/oder Überwachung einer Erkrankung, die sich durch die Expression oder abnormale Expression eines oder mehrerer Tumor-assoziierter Antigene auszeichnet. In einer Ausführungsform umfasst die Behandlung die Verabreichung einer pharmazeutischen Zusammensetzung. Vorzugsweise ist die Erkrankung Krebs, wobei der Begriff "Krebs" in nicht-begrenzender Weise Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

In einem Aspekt betrifft die Offenbarung ein Verfahren zur Diagnose einer Erkrankung, die sich durch die Expression oder abnormale Expression eines offenbarungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

In einem weiteren Aspekt betrifft die offenbarte Lehre ein Verfahren zur Bestimmung der Regression, des Verlaufs oder des Ausbruchs einer Erkrankung, die sich durch die Expression oder abnormale Expression eines offenbarungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, wodurch spezifische T-Lymphozyten identifiziert werden können.

In einem weiteren Aspekt betrifft die offenbarte Lehre ein Verfahren zur Behandlung, Diagnose oder Überwachung einer Erkrankung, die sich durch die Expression oder abnormale Expression eines offenbarungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel oder Stoff gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

In manchen Ausführungsformen erfolgen die offenbarten Verfahren zur Diagnose oder Überwachung einer Erkrankung, die sich durch die Expression oder abnormale Expression eines offenbarungsgemäß identifizierten Tumor-assoziierten Antigens auszeichnet, mit Hilfe von oder durch Nachweis von disseminierenden Tumorzellen oder von Tumormetastasen. Ein Nachweis disseminierender Tumorzellen kann beispielsweise im Blut, Serum, Knochenmark, Sputum, Bronchialaspirat und/oder Bronchiallavage erfolgen.

Die offenbarte Lehre betrifft auch ein Verfahren zur Behandlung eines Patienten mit einer Erkrankung, die sich durch die Expression oder abnormale Expression eines offenbarungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die vorliegende Lehre betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die vorliegende Offenbarung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die offenbarte Lehre betrifft auch ein Verfahren zur Behandlung einer Erkrankung, die sich durch die Expression oder abnormale Expression eines offenbarungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die offenbarungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Offenbarung eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NO: 3-5, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. Des weiteren betrifft die Offenbarung eine Nukleinsäure, die für ein Protein oder Polypeptid kodiert, das eine Aminosäuresequenz umfasst, ausgewählt aus der Gruppe bestehend aus SEQ ID NO: 10, 12-14 und 146-150, einem Teil oder Derivat davon.

In einem weiteren Aspekt betrifft die offenbarte Lehre Promotorsequenzen von offenbarungsgemäßen Nukleinsäuren. Diese können funktionell mit einem anderen Gen vorzugsweise in einem Expressionsvektor verbunden werden und somit die selektive Expression dieses Gens in entsprechenden Zellen gewährleisten.

In einem weiteren Aspekt betrifft die Erfindung ein rekombinantes Nukleinsäuremolekül, insbesondere DNA- oder RNA-Molekül, das eine offenbarungsgemäße Nukleinsäure umfasst.

Die vorliegende Lehre betrifft auch Wirtszellen, die eine offenbarungsgemäße Nukleinsäure oder ein rekombinantes Nukleinsäuremolekül, das eine offenbarungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die offenbarungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die offenbarte Lehre Oligonukleotide, die mit einer offenbarungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer offenbarungsgemäß identifizierten Nukleinsäure oder Teilen davon hybridisieren, können zum Auffinden von Nukleinsäuren verwendet werden, die zu der offenbarungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Offenbarung ein Protein, Polypeptid oder Peptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: 3-5, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform betrifft die Offenbarung ein Protein oder Polypeptid oder Peptid, das eine Aminosäuresequenz umfasst, ausgewählt aus der Gruppe bestehend aus SEQ ID NO: 10, 12-14 und 146-150, einem Teil oder Derivat davon.

In einem weiteren Aspekt betrifft die offenbarte Lehre ein immunogenes Fragment eines offenbarungsgemäß identifizierten Tumor-assoziierten Antigens. Das Fragment bindet vorzugsweise an einen menschlichen HLA-Rezeptor oder menschlichen Antikörper. Vorzugsweise umfasst ein offenbarungsgemäßes Fragment eine Sequenz von mindestens 6, insbesondere mindestens 8, mindestens 10, mindestens 12, mindestens 15, mindestens 20, mindestens 30 oder mindestens 50 Aminosäuren.

In diesem Aspekt betrifft die Offenbarung insbesondere ein Peptid, das eine Sequenz, ausgewählt aus der Gruppe bestehend aus SEQ ID NO: 17-19, 90-97, 100-102, 105, 106, 111-116, 120, 123, 124, 135-137, 139 und 142-150, einem Teil oder Derivat davon aufweist oder umfasst.

In einem weiteren Aspekt betrifft die offenbarte Lehre ein Mittel, das an ein offenbarungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellter Antikörper oder ein Fragment eines Antikörpers. Des weiteren betrifft die Offenbarung einen Antikörper, der selektiv an einen Komplex aus (i) einem offenbarungsgemäß identifizierten Tumor-assoziierten Antigen oder einem Teil davon und (ii) einem MHC-Molekül bindet, an das das offenbarungsgemäß identifizierte Tumor-assoziierte Antigen oder der Teil davon bindet, wobei der Antiköper nicht alleine an (i) oder (ii) bindet. Ein Antikörper kann ein monoklonaler Antikörper sein. In weiteren Ausführungsformen ist der Antikörper ein chimärer oder humanisierter Antikörper oder ein Fragment eines natürlichen Antikörpers.

Insbesondere betrifft die vorliegende Offenbarung ein solches Mittel, insbesondere einen Antikörper, das/der spezifisch an ein Peptid bindet, das eine Sequenz ausgewählt aus der Gruppe bestehend aus SEQ ID NO: 17-19, 90-97, 100-102, 105, 106, 111-116, 120, 123, 124, 135-137, 139 und 142-150, einem Teil oder Derivat davon aufweist oder umfasst.

Hinsichtlich Claudin-18 betrifft die Erfindung auch Mittel, insbesondere Antikörper, die spezifisch an eine Variante von Claudin-18 binden. In einer Ausführungsform bindet das Mittel, insbesondere ein Antikörper, spezifisch an die Variante Claudin-18A1 (SEQ ID NO: 118). In einer anderen Ausführungsform bindet das Mittel, insbesondere ein Antikörper, spezifisch an die Variante Claudin-18A2 (SEQ ID NO: 16). Solche spezifischen Antikörper können beispielweise durch Immunisieren mit den in Beispiel 4 beschriebenen Peptiden erhalten werden.

Des weiteren betrifft die Erfindung hinsichtlich Claudin-18 Mittel, insbesondere Antikörper, die spezifisch an eine Form von Claudin-18A2 binden, die ein bestimmtes Glykosylierungmuster aufweist. In einer Ausführungsform bindet das Mittel, insbesondere ein Antikörper, spezifisch an eine Form von Claudin-18A2, die an einer oder mehreren möglichen Glykosylierungsstellen nicht-glykosyliert ist. In einer anderen Ausführungsform bindet das Mittel, insbesondere ein Antikörper, spezifisch an eine Form von Claudin-18A2, die an einer oder mehreren möglichen Glykosylierungsstellen glykosyliert ist. Vorzugsweise betrifft eine derartige mögliche Glykosylierungsstelle eine oder mehrere Positionen, die aus der Gruppe ausgewählt sind, bestehend aus den Aminosäurepositionen 37, 38, 45, 116, 141, 146 und 205 von Claudin-18A2. Des weiteren betrifft eine derartige mögliche Glykosylierung vorzugsweise eine N-Glykosylierung.

Ein für eine Variante bzw. Form von Claudin-18 spezifisches Mittel, insbesondere ein für eine Variante bzw. Form von Claudin-18 spezifischer Antikörper, meint in diesem Zusammenhang, dass das Mittel bzw. der Antikörper stärker an die Variante bzw. Form für die es/er spezifisch ist, bindet als an eine andere Variante bzw. Form. Ein Mittel, insbesondere Antikörper, bindet stärker an eine erste Variante bzw. Form oder an ein erstes Epitop im Vergleich zu einer zweiten Variante bzw. Form oder einem zweiten Epitop, wenn es/er an die erste Variante bzw. Form oder an das erste Epitop mit einer Dissoziationskonstante (K_{D}) bindet, die geringer ist als die Dissoziationskonstante für die zweite Variante bzw. Form oder das zweite Epitop. Vorzugsweise ist die Dissoziationskonstante (K_{D}) für die Variante bzw. Form oder das Epitop, an die/das das Mittel, insbesondere ein Antikörper, spezifisch bindet mehr als 10-fach, vorzugsweise mehr als 20-fach, mehr bevorzugt mehr als 50-fach, noch mehr bevorzugt mehr als 100-fach und insbesondere mehr als 200-fach, 500-fach oder 1000-fach geringer als die Dissoziationskonstante (K_{D}) für die Variante bzw. Form oder das Epitop, an die/das das Mittel, insbesondere ein Antikörper, nicht spezifisch bindet. Vorzugsweise bindet ein Mittel, insbesondere ein Antikörper, nicht oder im wesentlichen nicht an die Variante bzw. Form oder das Epitop für die/das das Mittel, insbesondere der Antikörper, nicht spezifisch ist.

Die vorstehend beschriebenen Mittel, insbesondere Antikörper und Derivate davon wie hierin beschrieben, die an eine Variante bzw. Form von Claudin-18 spezifisch binden, sind auch für eine Verwendung in den erfindungsgemäßen Zusammensetzungen und Verfahren vorgesehen.

Des weiteren betrifft die Erfindung ein Konjugat zwischen einem offenbarungsgemäßen Mittel, das an ein offenbarungsgemäß identifiziertes Tumor-assoziiertes Antigen oder an einen Teil davon bindet, oder einem offenbarungsgemäßen Antikörper und einem therapeutischen oder diagnostischen Mittel oder Stoff. In einer Ausführungsform ist das therapeutische oder diagnostische Mittel ein Toxin.

In einem weiteren Aspekt betrifft die offenbarte Lehre einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

### Detaillierte Beschreibung der Erfindung

Vorliegend werden Gene beschrieben, die in Tumorzellen selektiv exprimiert oder aberrant exprimiert werden und Tumor-assoziierte Antigene darstellen.

Offenbarungsgemäß sind diese Gene und/oder deren Genprodukte und/oder ihre Derivate und/oder Teile bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist offenbarungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist offenbarungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann offenbarungsgemäß als einzelsträngiges oder doppelsträngiges und lineares oder kovalent kreisförmig geschlossenes Molekül vorliegen.

Die offenbarungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können offenbarungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist ein rekombinantes DNA-Molekül ein Vektor, gegebenenfalls mit einem Promotor, der die Expression einer Nukleinsäure, z.B. einer Nukleinsäure, die für ein offenbarungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein offenbarungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft offenbarungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird offenbarungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Offenbarung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Offenbarungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst offenbarungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die offenbarungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im Wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Offenbarungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen, Polypeptiden oder Peptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine, Polypeptide oder Peptide. Ferner erstreckt sich der Begriff "Derivat" auch auf alle funktionellen chemischen Äquivalente der Proteine, Polypeptide oder Peptide.

Ein Teil oder Fragment eines Tumor-assoziierten Antigens weist eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft offenbarungsgemäß den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Lehre ermöglicht auch die Isolierung von Proteinen, die an hier beschriebene Tumor-assoziierte Antigene binden, einschließlich Antikörper und zelluläre Bindepartner der Tumor-assoziierten Antigene.

Vorliegend werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Die offenbarte Lehre umfasst auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Offenbarungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Solche Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Offenbarungsgemäß bereitgestellt werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden. Offenbarungsgemäß umfasst sind auch sogenannte einzelkettige Antikörper.

Offenbarungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluorin und Gadolinium. Der Begriff "therapeutisch nützlicher Stoff' meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Der Begriff "Patient" bedeutet offenbarungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausfuhrungsform ist der Patient ein Mensch.

Der Begriff "Erkrankung" betrifft offenbarungsgemäß jeden pathologischen Zustand, bei dem Tumor-assoziierte Antigene exprimiert oder abnormal exprimiert werden. "Abnormale Expression" bedeutet erfindun offenbarungsgemäß gsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, wobei der Begriff "Krebs" erfindungsgemäß Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Sputum, Urin, Fäces oder anderen Körperflüssigkeiten.

Der Begriff "immunreaktive Zelle" bedeutet offenbarungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse 1-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse 1-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von gesunden Personen oder von einer anderen Spezies (z.B. Maus) kombiniert. Dies führt zu einer Vermehrung hochaffiner spezifischer cytotoxischer T-Lymphozyten, wenn die T-Lymphozyten aus einem Spenderorganismus kommen, der mit dem spezifischen Komplex bisher keinen Kontakt hatte. Der hochaffine T-Zell-Rezeptor aus diesen vermehrten spezifischen T-Lymphozyten wird kloniert. Wurden die hochaffinen T-Zellrezeptoren aus einer anderen Spezies kloniert, können diese in unterschiedlichem Ausmaß humanisiert werden. Durch Gentransfer z.B. mit retroviralen Vektoren werden solche T-Zell-Rezeptoren dann beliebig in T-Zellen von Patienten transduziert. Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer T-Zell-Antworten entstehen können. Eine effektive Immunantwort benötigt diese (Ossendorp et al., Immunol Lett. 74:75-9, 2000; Ossendorp et al., J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc Natl Acad Sci USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J Immunol. 167:787-96, 2001; Appella et al., Biomed Pept Proteins Nucleic Acids 1:177-84, 1995; Wentworth et al., Mol Immunol. 32:603-12, 1995).

Die offenbarungsgemäß beschriebenen pharmazeutischen Zusammensetzungen können auch als Vakzinen für die Immunisierung eingesetzt werden. Die Begriffe "Immunisierung" oder "Vakzinierung" bedeuten offenbarungsgemäß eine Erhöhung oder Aktivierung einer Immunreaktion gegenüber einem Antigen. Tiermodelle können zum Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vakzinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Nat. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Cytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren.

Offenbarungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex* vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Offenbarungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um offenbarungsgemäß Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die offenbarungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien, CpG und Cytokinen und gegebenenfalls andere therapeutische Wirkstoffe enthalten.

Die therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nicht-toxisches Material, das nicht mit der Wirkung des aktiven Bestandteils der pharmazeutischen Zusammensetzung wechselwirkt.

Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine offenbarungsgemäße pharmazeutische Zusammensetzung kann einen pharmazeutisch verträglichen Träger umfassen. Der Begriff "pharmazeutisch verträglicher Träger" betrifft offenbarungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Offenbarungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

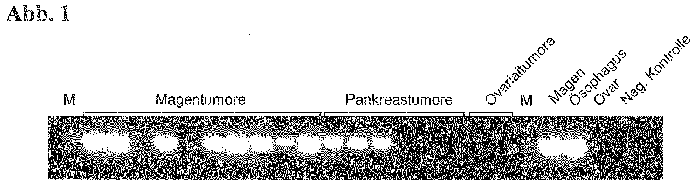

### Abb. 1. Claudin-18A2.1-Expression in Magen und Ösophagus sowie Magen- und Pankreastumoren

RT-PCR-Analyse mit Claudin-18A2.1-spezifischen Primern (SEQ ID NO: 39, 40) zeigte erfindungsgemäß in 8/10 Magentumor-Biopsien sowie in 3/6 Pankreastumor-Biopsien eine ausgeprägte Claudin-18A2.1-Expression. In Magen- und Ösophagus-Normalgeweben wurde ebenfalls eine deutliche Expression nachgewiesen. Im Gegensatz dazu wurde im Ovar und in Ovarialtumoren keine Expression detektiert.

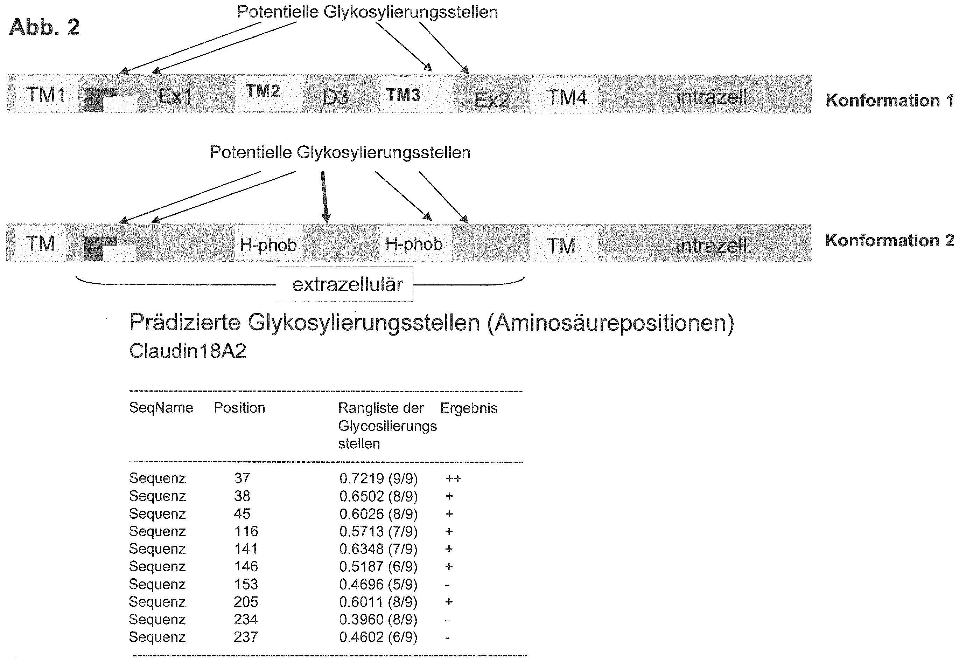

### Abb. 2. Schematische Darstellung von Claudin-18-Konformationen

Erfindungsgemäß kann das Claudin-18A2-Polypeptid in zwei Konformationen auf der Zelle vorliegen. In der Konformation 1 liegt das Protein als Membranmolekül mit 4 Transmembrandomänen (TM) vor und weist zwei separate, extrazellulär gelegene Domänen auf. In der Konformation 2 üben die beiden mittleren hyrophoben Bereiche (h-phob) keine Transmembrandomänen-Funktion aus. Dadurch sind in dieser Konformation im Vergleich zu der Konformation 1 zusätzliche Peptidregionen extrazellulär gelegen. Außerdem resultiert aus dieser Konformation eine zusätzliche N-Glykosylierungsstelle an Position 116 (dickerer Pfeil). Alle prädizierten Glykosylierungsdomänen sind im unteren Teil der Abbildung aufgeführt. Ex1: extrazelluläre Domäne 1, Ex2: extrazelluläre Domäne 2, TM: Transmembrandomäne, H-phob: extrazelluläre hydrophobe Region.

### Abb. 3. Quantitative Expression von Claudin-18, Variante A1

Claudin-18A1 ist in keinem Normalgewebe außer Lungen- und Magengewebe detektierbar. Claudin-18A1 ist in einer Vielzahl von Tumorgeweben stark exprimiert. Eine besonders starke Expression findet sich in Magentumoren, Lungentumoren, Pankreastumoren und Ösophagustumoren.

### Abb. 4. Quantitative Expression von Claudin-18, Variante A2

Claudin-18A2 ist in keinem Normalgewebe ausser Magengewebe detektierbar. Claudin-18A2 ist in einer Vielzahl von Tumorgeweben insbesondere Magentumoren, Lungentumoren, Pankreastumore und Ösophagustumoren exprimiert.

### Abb. 5. Verwendung Claudin-18A2-spezifischer Antikörper (extrazelluläre Domäne)

**A:** Färbung von Claudin-18A2-positiven Magentumorzellen (SNU-16, Methanol-fixiert) mit einem Antikörper, der durch Immunisierung mit einem Peptid (SEQ ID NO: 17) hergestellt wurde. Eine Membranfärbung tritt besonders stark in den Zell/Zell-Interaktionsbereichen auf. Das Protein aggregiert in fokalen Membranbereichen.
**B, C, D:** Nachweis der Spezifität des Antikörpes durch Kolokalisationsanalyse in Claudin-18A2-GFP-transfizierten 293T-Zellen. B: GFP-Fluoreszenz; C: anti-Claudin-18A2; D: Überlagerung.

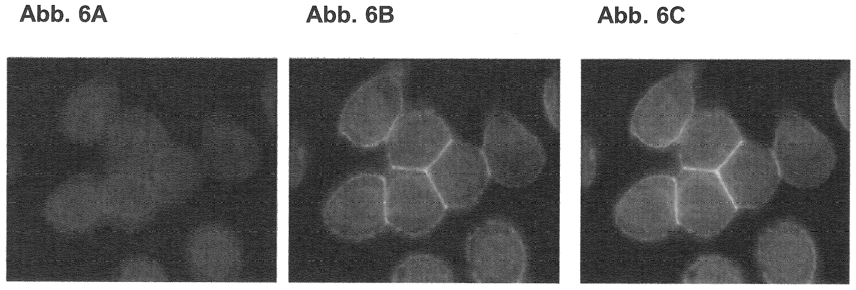

### Abb. 6. Verwendung Claudin-18A2-spezifischer Antikörper (extrazelluläre Domäne)

Membranfärbung von Claudin-18A2-positiven Magentumorzellen (SNU-16) mit einem Antikörper, der durch Immunisierung mit einem Peptid (SEQ ID NO: 113, N-terminal gelegene extrazelluläre Domäne) hergestellt wurde. Zur Gegenfärbung wurde ein monoklonaler Antikörper verwendet, der gegen E-Cadherin gerichtet ist. A: Claudin-18A2-Antikörper; B: anti E-Cadherin-Gegenfärbung; C: Überlagerung.

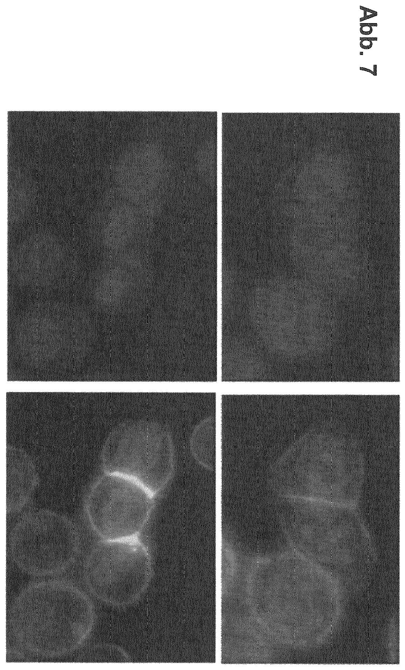

### Abb. 7. Verwendung von Antikörpern gegen die C-terminale extrazelluläre Domäne von Claudin-18

Linke Abbildungen: Membranfärbung von Claudin-18A2-positiven Magentumorzellen (SNU-16) mit einem Antikörper, der durch Immunisierung mit einem Peptid (SEQ ID NO: 116, C-terminal gelegene extrazelluläre Domäne) hergestellt wurde. Zur Gegenfärbung wurde ein monoklonaler Antikörper verwendet, der gegen E-Cadherin gerichtet ist (rechte Abbildungen).

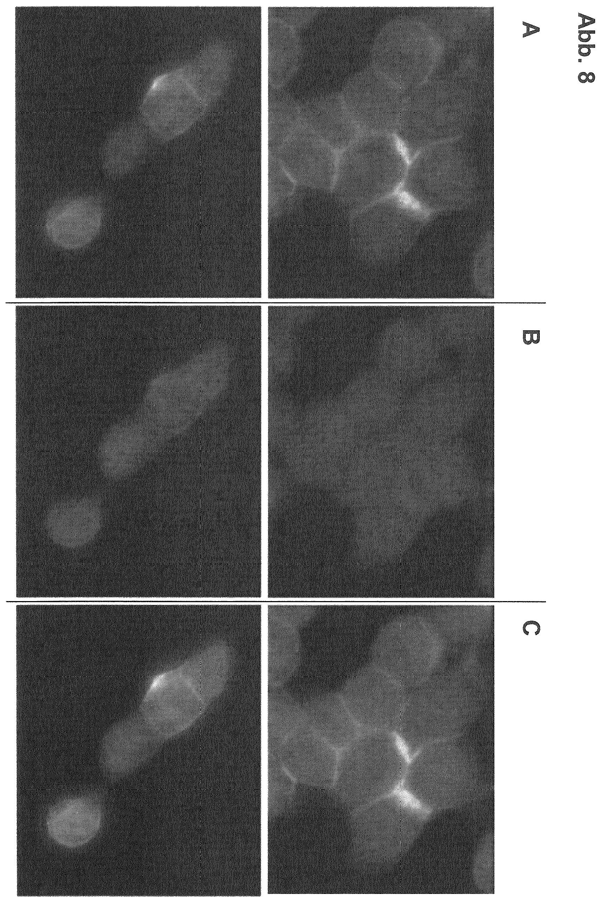

### Abb. 8. Verwendung Claudin-18A1-spezifischer Antikörper

Oben: schwache bis fehlende Färbung von Magentumorzellen (SNU-16; Claudin18A2-positiv) mit einem Antikörper, der durch Immunisierung mit einem Claudin-18A1-spezifischen Peptid (SEQ ID NO: 115) hergestellt wurde. A: anti-E-Cadherin; B: anti-Claudin-18A1; C: Überlagerung.

Unten: Nachweis der Spezifität des Antikörpes durch Kolokalisationsanalyse in Claudin-18A1-GFP-transfizierten 293T-Zellen. A: GFP-Fluoreszenz; B: anti-Claudin-18A1; C: Überlagerung.

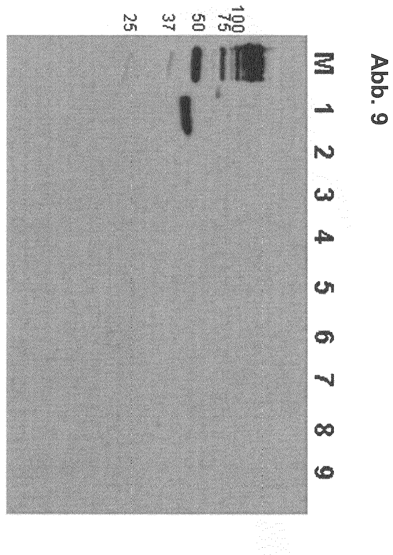

### Abb. 9. Nachweis von Claudin-18A2 im Western-Blot.

Western-Blot mit Lysaten aus verschiedenen gesunden Geweben mit einem Claudin-18A2-spezifischen Antikörper, gerichtet gegen das Epitop mit SEQ ID NO: 17. 1: Magen-; 2: Testis-; 3: Haut-; 4: Brust-; 5: Leber-; 6: Kolon-; 7: Lunge-; 8: Niere-; 9: Lymphknoten-Normalgewebe.

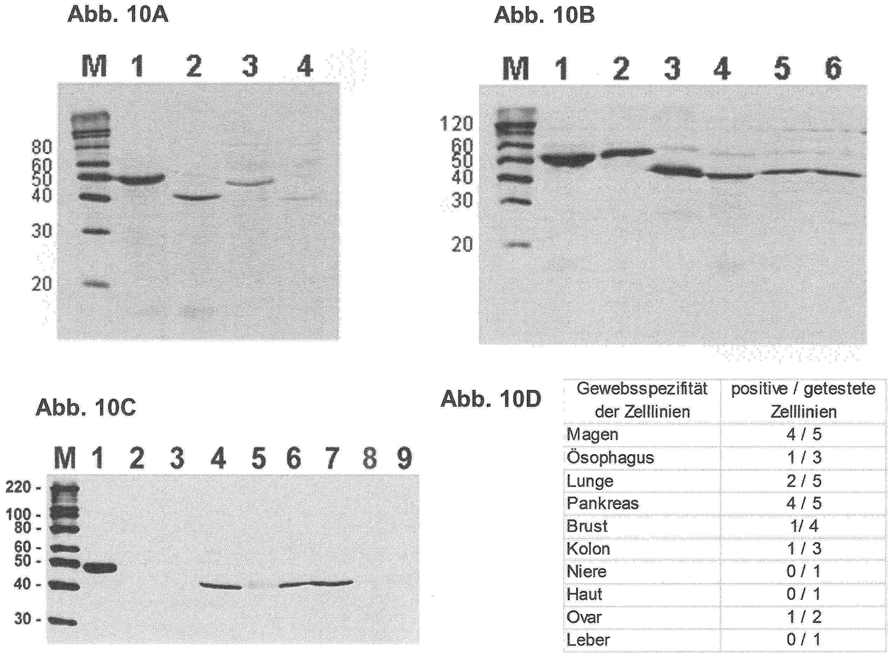

### Abb. 10. Western-Blot von Claudin-18A2 mit Proben aus Magen und Magentumoren sowie verschiedenen Tumorzelllinien.

Lysate aus Magen und Magentumoren (A, B) und Tumorzelllinien (C, D) wurden geblottet und mit einem Claudin-18A2-spezifischen Antikörper gegen das Epitop mit SEQ ID NO: 17 getestet. Magentumore weisen eine geringer glykosylierte Form von Claudin-18A2 auf. Eine PNGase F-Behandlung von Magenlysaten führt zur Bildung der niedrigglykosylierten Form.
**A:** 1: Magen-Normalgewebe #A; 2: Magentumor #A; 3: Magen-Normalgewebe #B; 4: Magentumor #B
**B:** 1: Magen-Normalgewebe #A; 2: Magen-Normalgewebe #B; 3: Magen-Normalgewebe #B + PNGase F; 4: Magentumor #C; 5: Magentumor #D; 6: Magentumor #D + PNGase F
**C:** 1: Magen-Normalgewebe; 2: MDA-MB-231; 3: SK-MEL-37; 4: AGS; 5: SNU-1; 6: SNU-16; 7: EFO27; 8: TOV-112D; 9: OVCAR. Zu beachten ist, dass die Tumorzelllinien die deglykosylierte Variante von Claudin-18A2 exprimieren.
**D:** Tabellarische Zusammenfassung der Western-Blot-Daten für eine Auswahl von Zelllinien, die mit dem Claudin-18A2-spezifischen Antikörper getestet wurden.

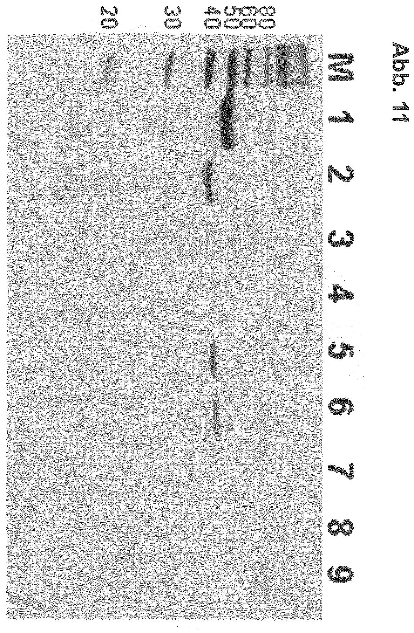

### Abb. 11. Expression von Claudin-18 in Lungentumoren

Entsprechend zu Abb. 30 erfolgte ein Nachweis von niedrigglykosylierten Claudin-18A2-Varianten in Lungentumoren. 1: Magen-Normalgewebe; 2: Magentumor; 3-9: Lungentumore.

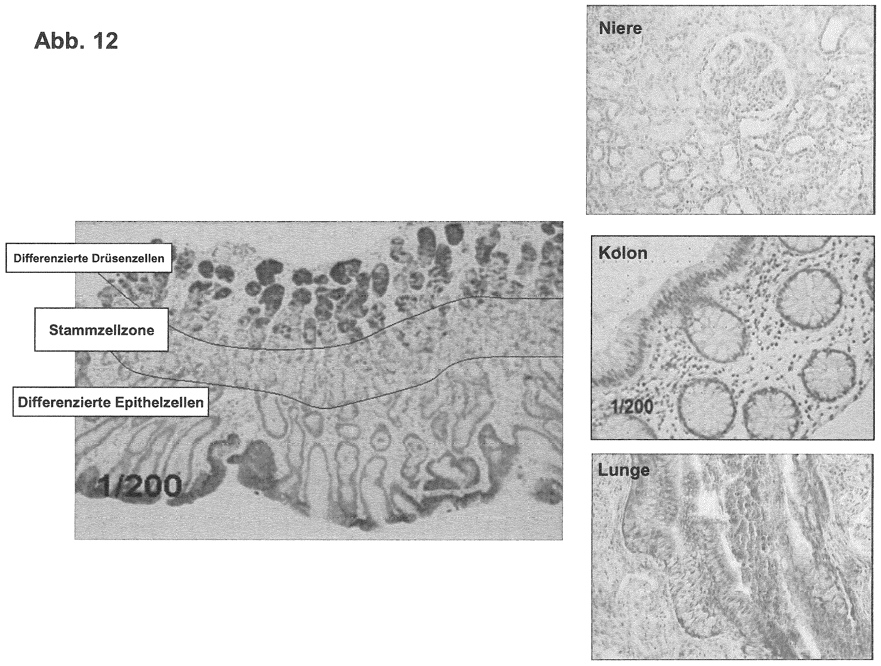

### Abb. 12. Immunhistochemische Analyse von Claudin-18 mit Claudin-18A2-spezifischen Antikörpern in Normalgeweben

In der Magenschleimhaut werden lediglich differenzierte epitheliale Zellen an der Öffnung wie auch am Boden der Drüsen gefärbt. Claudin-18A2 ist in Stammzellen des Magens nicht nachweisbar. Alle anderen von uns untersuchten Normalgewebe exprimieren dieses Gen ebenfalls nicht, wie beispielhaft für Niere, Lunge und Kolon dargestellt.

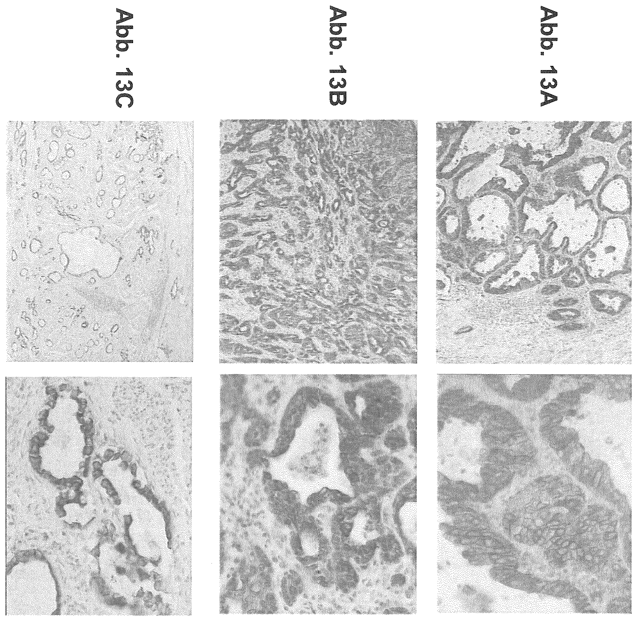

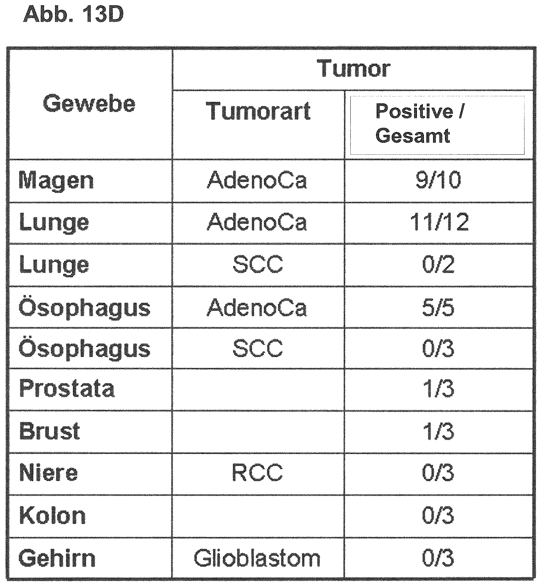

### Abb. 13. Resultate der Immunhistologie mit Claudin-18A2-spezifischem polyklonalen Antiserum

**A:** Beispiele für spezifische Färbungen von Lungentumor-Geweben. Zu beachten ist, dass das normale Lungengewebe, welches die Variante Claudin-18A1 exprimiert, durch das Claudin-18A2-spezifische Antiserum nicht erkannt wird.
**B:** Beispiele für spezifische Tumorfärbung von Ösophagustumoren. Zu beachten ist, dass gesunde Zellen in der Umgebung nicht gefärbt werden.
**C:** Beispiele für spezifische Tumorfärbung von Magentumorepithelien. Auch hier werden gesunde Zellen in der Umgebung nicht gefärbt.
**D:** Beispeilhafte tabellarische Zusammenfassung immunhistochemischer Färbedaten mit Claudin-18A2-spezifischen Antikörpern. AdenoCa: Adenokarzinom; SCC: Plattenepithelkarzinom; RCC: Nierenzellkarzinom.

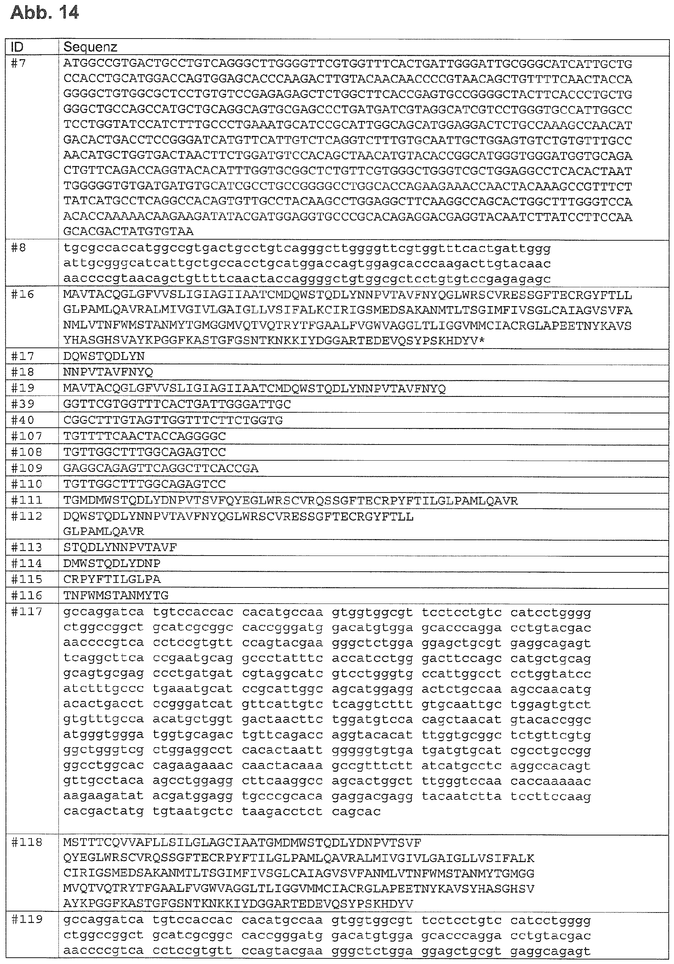

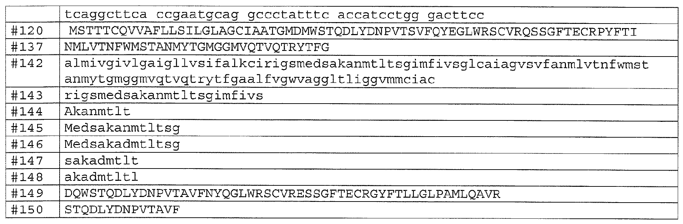

### Abb. 14. Sequenzen

Gezeigt sind die Sequenzen, auf die hierin verwiesen wird.

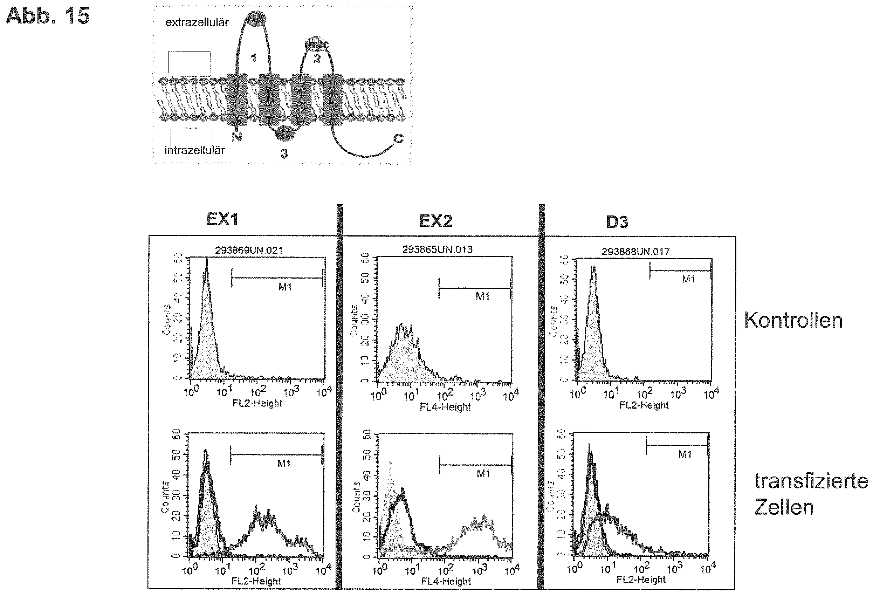

### Abb. 15. Ermittlung extrazellulärer Bereiche von Claudin-18A2

Drei Konstrukte wurden hergestellt, die jeweils eine Marker-Sequenz (myc- oder HA-Tag) in einer der Domänen EX1 (= extrazelluläre Domäne 1), EX2 (= extrazelluläre Domäne 2), oder D3 (= Domäne 3) trugen (oben). Diese wurden in Zelllinien transfiziert und daraufhin getestet, ob ein gegen diese Markersequenzen gerichteter Antikörper an nichtpermeabilisierte Zellen bindet. Dies erfordert, dass der entsprechende Bereich des Proteins topologisch extrazellulär ist. In der Durchflusszytometrie erwies sich, dass alle drei Bereiche des Moleküls für die Antikörper zugänglich sind (unten).

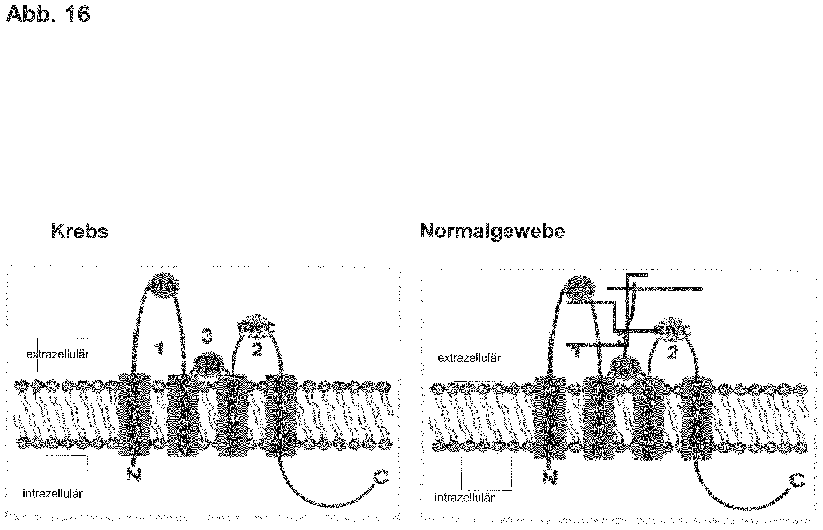

### Abb. 16. Claudin-18A2-Membrantopologie

Entsprechend unserer Daten kann Claudin-18A2 in der Konformation 2 vorliegen, wobei die inneren beiden hydrophoben Domänen nicht integral durch die Zellmembran hindurchführen. Dadurch sind größere Bereiche dieses Moleküls extrazellulär. Hier befinden sich auch Glykosylierungsdomänen, die nach unseren Daten in Magennormalgewebe glykosyliert sind, in Tumoren aber nicht. Es entstehen somit rein tumorgewebsspezifische Epitope.

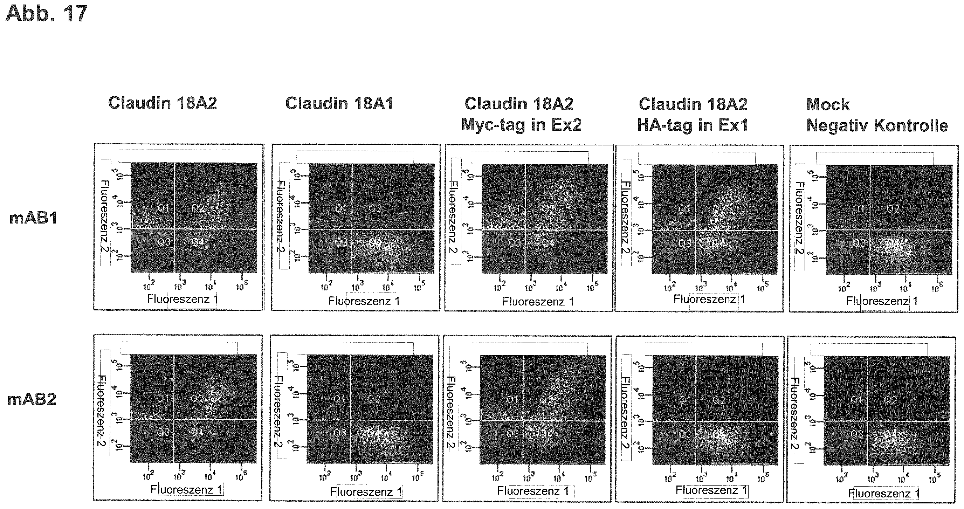

### Abb. 17. FACS-Analyse zur Bestimmung der extrazellulären Lokalisation von Claudin 18

Die Abbildung zeigt durchflusszytometrische Analysen an nichtpermeabilisierten Zellen, die mit Voll-Längen Claudin-18A1, Claudin-18A2 und Mock sowie Abschnitten aus Claudin-18A2 transfiziert wurden. Erkennbar ist, dass die Antikörper mAB1 und mAB2 spezifisch Claudin-18A2 (linke Spalte) und die extrazelluläre Domäne 2 (Ex2, 3. Spalte) an der Zelloberfläche erkennen, während Claudin-18A1 (2. Spalte) und die Negativ-Kontrolle (letzte Spalte) negativ sind. Der Antikörper mAB1 bindet, im Gegensatz zu mAB2, auch spezifisch die extrazelluläre Domäne 1 (Ex1, 4. Spalte).

### Beispiele:

### Material und Methoden

Die Begriffe "*in. silico*", "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini werden, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von neuen Tumor-assoziierten Genen

Zwei *in silico* Strategien nämlich GenBank-Schlagwort-Suche und der cDNAxProfiler wurden kombiniert. Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in bestimmten Geweben (Wheeler et al., Nucleic Acids Research 28:10-14, 2000).

Durch Suchabfragen mit Schlagworten wie beispielsweise "colon-specific gene", " stomachspecific gene", oder "kidney-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.

Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht Organ-spezifischen Normalgeweben hatten. Für dies Beurteilung wurde auch berücksichtigt, dass es falsch-annotierte cDNA-Banken in der öffentlichen Domäne gibt (Scheurle et al., Cancer Res. 60: 4037-4043, 2000) (www.fau.edu/cmbb/publications/cancergenes6.htm). Als zweites Datamining-Verfahren wurde der cDNA xProfiler des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt, Pools von in Datenbanken abgelegten Transkriptomen durch logische Operatoren in Beziehung zueinander zu setzen. Wir haben einen Pool A definiert, dem beispielsweise alle aus Colon hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Colon hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthern-Studien unterzogen, sowie durch eine Literaturrecherche abgesichert.

Dieses kombinierte Datamining schließt alle etwa 13 000 Volllänge-Gene in der öffentlichen Domäne ein und prädiziert aus diesen Gene mit potentieller Organ-spezifischer Expression.

Alle anderen Gene wurden zunächst durch spezifische RT-PCR in Normalgeweben evaluiert. Alle GOI, die sich als in nicht Organ-spezifischen Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als in Tumorzellen aktiviert.

### RNA-Extraktion, Herstellung von poly-d(T) geprimter cDNA und konventionelle RT-PCR Analyse

Gesamt-RNA aus nativem Gewebematerial wurde unter Verwendung von Guanidiumisothiocyanat als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.

Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.

Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.

Die Primer wurden so ausgewählt, dass sie in 2 verschiedenen Exons liegen, und die Beseitigung der Interferenz durch kontaminierende genomische DNA als Grund für falsch positive Resultate wurde durch Testen von nicht revers transkribierter DNA als Matrize bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min).

20µl dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet.

### Claudin-18A1 (64°C)

| | |
|---|---|
| Sense: 5'-GAGGCAGAGTTCAGGCTTCACCGA-3' | (SEQ ID NO: 109) |
| Antisense: 5'-TGTTGGCTTTGGCAGAGTCC-3' | (SEQ ID NO: 110) |

### Claudin-18A2 (68°C)

| | |
|---|---|
| Sense1: 5'-GGTTCGTGGTTTCACTGATTGGGATTGC-3' | (SEQ ID NO: 39) |
| Antisense1: 5'-CGGCTTTGTAGTTGGTTTCTTCTGGTG-3' | (SEQ ID NO: 40) |
| Sense2: 5'-TGTTTTCAACTACCAGGGGC-3' | (SEQ ID NO: 107) |
| Antisense2: 5'-TGTTGGCTTTGGCAGAGTCC-3' | (SEQ ID NO: 108) |

### Herstellung von Random-Hexamer-geprimter cDNA und quantitative Real-Time-PCR

Die Expression mehrere Gene wurde mittels Real-Time-PCR quantifiziert. Dabei wurden die PCR-Produkte mit SYBR-Green als interkalierendem Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green-Fluoreszenz als Folge der spezifischen Amplifikation mittels GOI-spezifischen Primern nach jedem PCR-Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression wurde nach Normalisierung der Proben gegen 18s RNA als sog. Housekeeping-Gen mittels der ΔΔ-Cₜ Methode (PE Biosystems, USA) ermittelt. Die Reaktionen wurden in Duplex-Ansätzen durchgeführt und in Triplikaten bestimmt. Verwendet wurde der QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer 300nM, antisense-Primer 300nM; initiale Denaturierung 95°C 15 min; 95°C 30 sec; Annealing 30 sec; 72°C 30 sec; 40 Zyklen. Die Sequenzen der verwendeten Primer sind in den jeweiligen Beispielen aufgeführt.

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA-Vektor zu klonieren. Die Sequenzierung wurde durch einen kommerziellen Service durchgeführt. Die Sequenzen wurden mittels gängiger Prädiktionsprogramme und Algorithmen analysiert.

### Western-Blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%eigen SDS Lösung lysiert. Das SDS denaturiert dabei die im Lysat enthaltenen Proteine. Die Lysate eines experimentellen Ansatzes werden abhängig von der erwarteten Proteingröße auf 8-15 %igen denaturierenden Polyacrylamidgelen (enthalten 1% SDS) der Größe nach elektrophoretisch aufgetrennt (SDS-Polyacrylamid-Gelelektrophorese, SDS-PAGE). Anschließend werden die Proteine durch das semi-dry Elektroblot Verfahren (Biorad) auf Nitrozellulose-Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

Die Analyse von Proteinmodifikationen erfolgt in der Regel im Western-Blot. Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate aus Geweben oder Zellen vor der Denaturierung durch SDS mit O- oder N-Glykosidasen inkubiert (nach Angaben des jeweiligen Herstellers, z.B. PNgase, Endoglykosidase F, Endoglykosidase H, Roche Diagnostics). Anschließend erfolgt ein Western-Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Mit Algorithmen und Prädiktionsprogrammen kann die genaue Position der glykosylierten Aminosäure prädiziert werden.

### Immunfluoreszenz

Es werden Zellen etablierter Zelllinien benutzt, die entweder das Zielprotein endogen synthetisieren (Nachweis der RNA in der RT-PCR oder des Proteins im Western-Blot) oder aber vor der IF mit Plasmid-DNA transfiziert worden sind. Zur Transfektion von Zelllinien mit DNA sind die verschiedensten Methoden (z.B. Elektroporation, Liposomen-basierte Transfektion, Calciumphosphatpräzipitation) gut etabliert (z.B. Lemoine et al. Methods Mol. Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen und kurze Peptidsequenzen von 6-12 Aminosäuren, für die hoch affine und spezifische Antikörper zur Verfügung stehen. Zellen, die das Zielprotein synthetisieren, werden mit Paraformaldehyd, Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und/oder (3) den Zelltyp in einem Gewebe (Tumor, gesunde Zellen) zu definieren, in dem das Zielprotein nachweisbar ist.
Je nach dem individuellen Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Die Immunhistochemie (IHC) an spezifischen Gewebeproben dient dem Proteinnachweis im entsprechenden Gewebe. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR-Analysen bekannt ist. Eine Hintergrundkontrolle ist immer mitzuführen.

Fixierte Gewebe (z.B. Fixation mit aldehydhaltigen Substanzen, Formaldehyd, Paraformaldehyd oder in alkoholischen Lösungen) oder schockgefrorene Gewebestücke mit einer Dicke von 1-10µm werden auf einem Glasträger aufgebracht. Paraffineingebettete Proben werden z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der von Farbsubstraten, die von dem gebundenen Enyzmen umgesetzt werden (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Immunisierung

(Siehe auch Monoclonal Antibodies: A Practical Approach by Philip Shepherd, Christopher Dean ISBN: 0-19-963722-9; Antibodies: A Laboratory Manual by Ed Harlow, David Lane ISBN: 0879693142; Using Antibodies: A Laboratory Manual: Portable Protocol NO. by Edward Harlow, David Lane, Ed Harlow ISBN: 0879695447).
Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.
Die Immunisierung der Tiere erfolgt in der Regel über eines von vier gut etablierten Verfahren, wobei auch andere Verfahren verfügbar sind. Immunisiert werden kann dabei mit Peptiden, die für das Zielprotein spezifisch sind, dem gesamten Protein oder mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro-Verfahren synthetisiert und diese Peptide zur Immunisierung verwendet. In der Regel erfolgen 3 Immunisierungen mit einer Konzentration von 5-1000 µg/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, wobei die Aufreinigung dabei in der Regel über standardisierte chromatografische Methoden erfolgt. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst-Fusionsproteine). Eine Vielzahl von Protokollen finden sich z.B. in den "Current Protocols in Molecular Biology", John Wiley & Sons Ltd., Wiley InterScience.
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend werden 5-100 µg DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et al., Hybrid Hybridomics 21: 287-293, 2002).

### Qualitätskontrolle des polyklonalen Serums bzw. Antikörpers

Zum Spezifitätsnachweis eignen sich am besten auf Zellkultur-basierende Tests mit anschließendem Western-Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter der Kontrolle eines starken eukaryontischen Promotors steht (z.B. Cytomegalovirus-Promotor). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgenspezifische RT-PCR). Zur Kontrolle werden im Experiment im Idealfall homologe Gene mit transfiziert, um im folgenden Western-Blot die Spezifität des analysierten Antikörpers nachweisen zu können.

Im anschließenden Western-Blot werden Zellen aus Zellkultur oder Gewebeproben, die das Zielprotein enthalten könnten, in einer 1%eigen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid-Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

Zur Bestätigung der im in silico-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western-Blot) oder aber mit Plasmid-DNA transfiziert worden sind. Zur Transfektion von Zelllinien mit DNA sind die verschiedensten Vefahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz den Nachweis, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zur Immunfluoreszenz kann durch Verwendung von z.B. Propidiumiodid oder Trypanblau zwischen toten und lebenden Zellen unterschieden werden und damit falsch positive Ergebnisse vermieden werden.

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin, pH 2,7 eluiert und das Eluat sogleich in 2 M TRIS, pH 8 neutralisiert. Die so gereinigten Antikörper konnten dann zur spezifischen Detektion der Zielproteine sowohl durch Westernblotting als auch durch Immunfluoreszenz eingesetzt werden.

### Herstellung von GFP-Transfektanten

Für die Immunfluoreszenz-Mikroskopie von heterolog exprimierten Tumor-assoziierten Antigenen wurde der komplette ORF der Antigene in pGFP-C1- und pGFP-N3-Vektoren (Clontech) kloniert. Auf Objekträgern kultivierte CHO- und NIH3T3-Zellen wurden mit den entsprechenden Plasmidkonstrukten unter Verwendung von Fugene-Transfektionsreagenz (Roche) nach Herstellerangaben transfiziert und nach 12-24h mittels Immunfluoreszenz-Mikroskopie analysiert.

### Durchflusszytometrie

Durchflusszytometrische Messungen wurden gemäß bekannter Verfahren durchgeführt (z. B. Robinson (Editor) Handbook of flow cytometry methods. Wiley-Liss, New York, 1993).

### Beispiel 1: Identifizierung von Claudin-18A1- und Claudin-18A2-Spleißvarianten als diagnostische und therapeutische Krebs-Targets

Das Claudin-18-Gen kodiert für ein Oberflächenmembranmolekül mit 4 hydrophoben Bereichen. Entsprechend der Prädiktionsprogramme (TMHMM, TMPred) und der für viele andere Mitglieder dieser Familie beschriebenen Topologie zufolge besitzt Claudin-18 vier Transmembran- und somit zwei extrazelluläre Domänen EX1 und EX2, deren extrazelluläre Lokalisation (Konformation 1) in Abb. 2 dargestellt ist. Die Domäne D3, die zwischen den beiden extrazellulären Epitopen lokalisiert ist, ist in der Literatur für Claudin-18 und andere Mitglieder dieser Familie als intrazellulär beschrieben und wird mit herkömmlichen Prädiktionsprogrammen auch so prädiziert. Der N- wie auch C-Terminus sind intrazellulär. Niimi und Kollegen (Mol. Cell. Biol. 21:7380-90, 2001) beschrieben zwei Spleißvarianten des Maus- und humanen Claudin-18, die als selektiv in Lungengewebe (Claudin-18A1) bzw. in Magengewebe (Claudin-18A2) exprimiert beschrieben wurden. Diese Varianten unterscheiden sich im N-Terminus.

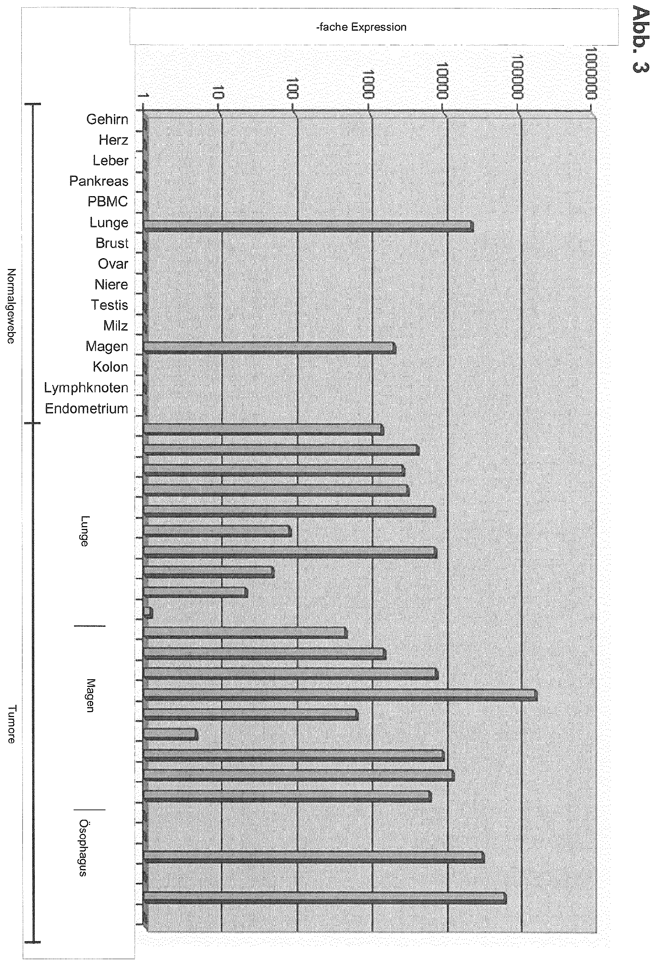

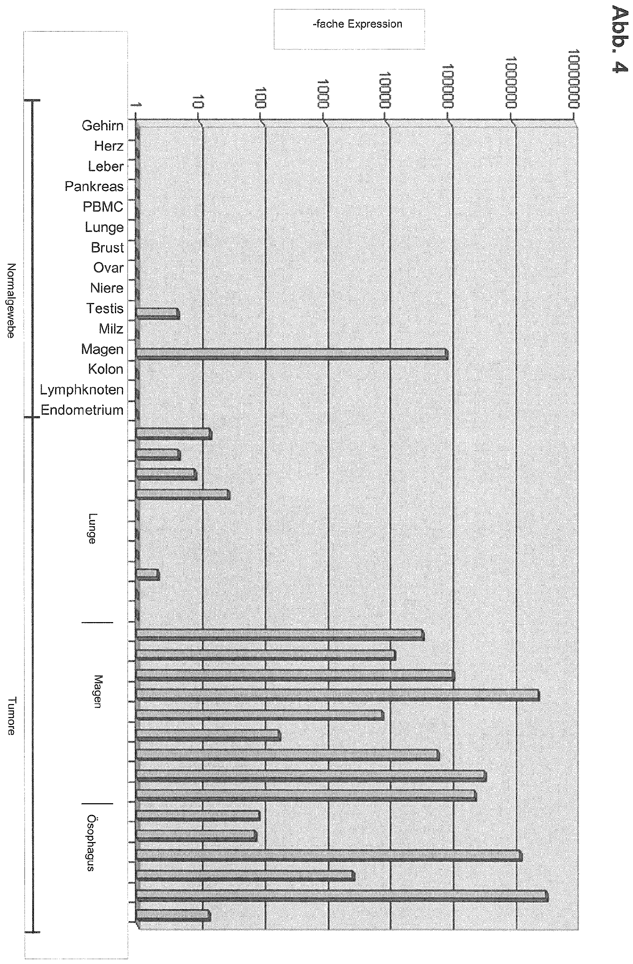

Erfindungsgemäß wurde untersucht, inwieweit die Spleißvarianten Claudin-18A2 (SEQ ID NO: 7) und Claudin-18A1 (SEQ ID NO: 117) sowie ihre jeweiligen Translationsprodukte (SEQ ID NO: 16 und 118) als Marker bzw. therapeutische Zielstrukturen für Tumoren genutzt werden können. Es wurde eine quantitative PCR etabliert, die zwischen beiden Varianten unterscheiden kann, indem A1-spezifische (SEQ ID NO: 109, 110) bzw. A2-spezifische (SEQ ID NO: 107, 108) Primerpaare ausgewählt wurden. Die Spleißvariante A2 wurde zusätzlich mit einem zweiten Primerpaar in einer konventionellen PCR getestet (SEQ ID NO: 39, 40). Für die Variante A1 ist beschrieben, dass sie nur in gesundem Lungengewebe aktiv ist. Jedoch stellten wir erfindungsgemäß überraschenderweise fest, dass die Variante A1 auch in der Magenschleimhaut aktiv ist (Abb. 3). Magen und Lunge sind die einzigen Normalgewebe, die eine signifikante Aktivierung aufweisen. Alle anderen Normalgewebe sind negativ für Claudin-A1. Bei der Untersuchung von Tumoren wurde überraschend festgestellt, dass Claudin-A1 in einer Vielzahl von Tumorgeweben stark aktiviert ist. Ein besonders starke Expression findet sich in Magentumoren, Lungentumoren, Pankreastumoren, Ösophagustumoren (Abb. 3), HNO-Tumoren und Prostatatumoren. Die Expressionsspiegel von Claudin-A1 in HNO-, Prostata-, Pankreas- und Ösophagustumoren sind 100-10000 höher als die Spiegel in den korrespondierenden Normalgeweben. Für die Untersuchung der Claudin-A2-Spleißvariante wurden Oligonukleotide verwendet, die spezifisch die Amplifikation dieses Transkripts ermöglichen (SEQ ID NO: 39, 40 bzw. 107, 108). Die Untersuchung ergab, dass die Spleißvariante A2 in keinem der mehr als 20 untersuchten Normalgewebe außer in Magenschleimhaut und in geringem Ausmaß auch Testisgewebe exprimiert wird (Abb. 4). Wir stellten fest, dass wie die Variante A1 auch die Variante A2 in vielen Tumoren aktiviert ist (Abb. 4). Hierzu zählen Magentumore, Pankreastumore, Ösophagustumore und Lebertumore. Obwohl sich in gesunder Lunge keine Aktivierung von Claudin-18A2 nachweisen lässt, wurde überraschend festgestellt, dass ein Teil der Lungentumoren die Spleißvariante A2 exprimieren.

**Tabelle 1A. Expression von Claudin-18A2 in Normal- und Tumor-Geweben**

| **Normalgewebe** | **Expression** |
|---|---|
| Gehirn | - |
| Cerebellum | - |
| Myokard | - |
| Skelettmuskel | - |
| Endometrium | - |
| Magen | +++ |
| Kolon | - |
| Pankreas | - |
| Niere | - |
| Leber | - |
| Testis (Hoden) | + |
| Thymus | - |
| Brust | - |
| Ovar | - |
| Uterus | - |
| Haut | - |
| Lunge | - |
| Schilddrüse | - |
| Lymphknoten | - |
| Milz | - |
| PBMC | - |
| Ösophagus | - |

| **Tumortyp** | **Expression** |
|---|---|
| Kolon | - |
| Pankreas | ++ |
| Ösophagus | ++ |
| Magen | +++ |
| Lunge | ++ |
| Brust | - |
| Ovar | - |
| Endometrium | n.u. |
| HNO | ++ |
| Niere | - |
| Prostata | - |

**Tabelle 1B. Expression von Claudin-18A1 in Normal- und Tumor-Geweben**

| | |
|---|---|
| **Normalgewebe** | **Expression** |
| Gehirn | - |
| Cerebellum | - |
| Herzmuskel | - |
| Muskel | - |
| Endometrium | - |
| Magen | +++ |
| Kolon | - |
| Pankreas | - |
| Niere | - |
| Leber | - |
| Testis | + |
| Thymus | - |
| Brust | - |
| Ovar | - |
| Uterus | - |
| Haut | - |
| Lunge | +++ |
| Schilddrüse | - |
| Lymphknoten | - |
| Milz | - |
| PBMC | - |
| Ösophagus | - |

| **Tumortyp** | **Expression** |
|---|---|
| Kolon | - |
| Pankreas | ++ |
| Ösophagus | ++ |
| Magen | +++ |
| Lunge | ++ |
| Brust | + |
| Ovar | n.u |
| Endometrium | n.u. |
| HNO | ++ |
| Niere | - |
| Prostata | ++ |

Auch die konventionelle PCR als unabhängige Kontrolluntersuchung bestätigte die Resultate der quantitativen PCR. Hierzu wurden Oligonukleotide (SEQ ID NO: 39, 40) verwendet, die eine spezifische Amplifikation der Spleißvariante A2 erlauben. Erfindungsgemäß wurde gezeigt, dass die meisten Magentumore und die Hälfte der getesteten Pankreastumore eine starke Expression dieser Spleißvariante aufweisen (Abb. 1). Dagegen ist eine Expression mit konventioneller PCR in anderen Geweben nicht nachweisbar. Insbesondere findet sich in wichtigen Normalgeweben wie Lunge, Leber, Blut, Lymphknoten, Brust und Niere keine Expression (Tab. 1). Hiermit stellen die Spleißvarianten erfindungsgemäß hochspezifische molekulare Marker für Tumoren des oberen Magendarmtraktes wie auch Lungentumoren, HNO-Tumoren, Prostatatumoren und ihre Metastasen dar. Diese molekulare Marker können erfindungsgemäß zum Nachweis von Tumorzellen genutzt werden. Die Detektion der Tumoren kann erfindungsgemäß mit den genannten Oligonukleotiden (SEQ ID NO: 39, 40, 107-110) erfolgen. Als Oligonukleotide eignen sich insbesondere Primerpaare, von denen mindestens einer unter stringenten Bedingungen an einen 180 Basenpaare langen Abschnitt des Transkripts bindet, der spezifisch für die eine (SEQ ID NO: 8) oder andere Spleißvariante (SEQ ID NO: 119) ist.

Diese Genprodukte sind attraktive therapeutische Zielstukturen, da durch ihr Fehlen in den meisten toxizitätsrelevanten Organen an diesen keine Nebenwirkungen zu erwarten sind, während die starke Aktivierung in Zellen der genannten Krebsarten eine gute Bindung an diese und Vermittlung ensprechender zellschädigender Effekte erwarten lässt.

Um diese Daten auf Proteinebene zu bestätigen, wurden Claudin-spezifische Antikörper bzw. Immunseren durch Immunisierung von Tieren generiert.
Die N-terminale extrazelluläre Domäne EX1 unterscheidet sich in der Sequenz bei den beiden Spleißvarianten A1 und A2 (SEQ ID NO: 111 für A1 und SEQ ID NO: 112 für A2). Die C-terminale extrazelluläre Domäne EX2 ist für beide Varianten identisch (SEQ ID NO: 137). Bisher sind noch keine Antikörper beschrieben, die an die extrazelluläre Domänen von Claudin-18 binden. Auch wurden bisher keine Antikörper beschrieben, die spezifisch zwischen A1- und A2-Varianten unterscheiden können. Erfindungsgemäß wurden für die Immunisierung zur Generierung von Antikörpern extrazellulär gelegene Peptidepitope und Proteinfragemente ausgewählt, die spezifisch für die Variante A1 oder A2 sind bzw. in beiden Varianten vorkommen. Unter anderem wurden zur Immunisierung folgende Peptide für die Herstellung von Antikörpern ausgewählt:
SEQ ID NO: 17: DQWSTQDLYN (N-terminal-extrazelluläre Domäne, A2-spezifisch, Bindung unabhängig von Glykosylierung)
SEQ ID NO: 18: NNPVTAVFNYQ (N-terminal-extrazelluläre Domäne, A2-spezifisch, Bindung hauptsächlich an unglykosylierte Form, N37)
SEQ ID NO: 113: STQDLYNNPVTAVF (N-terminal-extrazelluläre Domäne, A2-spezifisch, Bindung nur an nicht glykosylierte Form, N37)
SEQ ID NO: 114: DMWSTQDLYDNP (N-terminal-extrazelluläre Domäne, A1-spezifisch)
SEQ ID NO: 115: CRPYFTILGLPA (N-terminal-extrazelluläre Domäne, hauptsächlich spezifisch für A1)
SEQ ID NO: 116: TNFWMSTANMYTG (C-terminal-extrazelluläre Domäne, erkennt sowohl A1 als auch A2).

Wir konnten unter anderem Antikörper generieren, die selektiv die N-terminale Domäne der Spleißvariante Claudin-18-A1 erkennen, aber nicht die A2-Variante (Abb. 8). Durch Nutzung von Epitopen für Immunisierungen, welche in der C-terminal-extrazellulären Domäne liegen, die identisch in beiden Spleißvarianten ist, konnten wir Antikörper herstellen, die beide Varianten erkennen (Abb. 7).

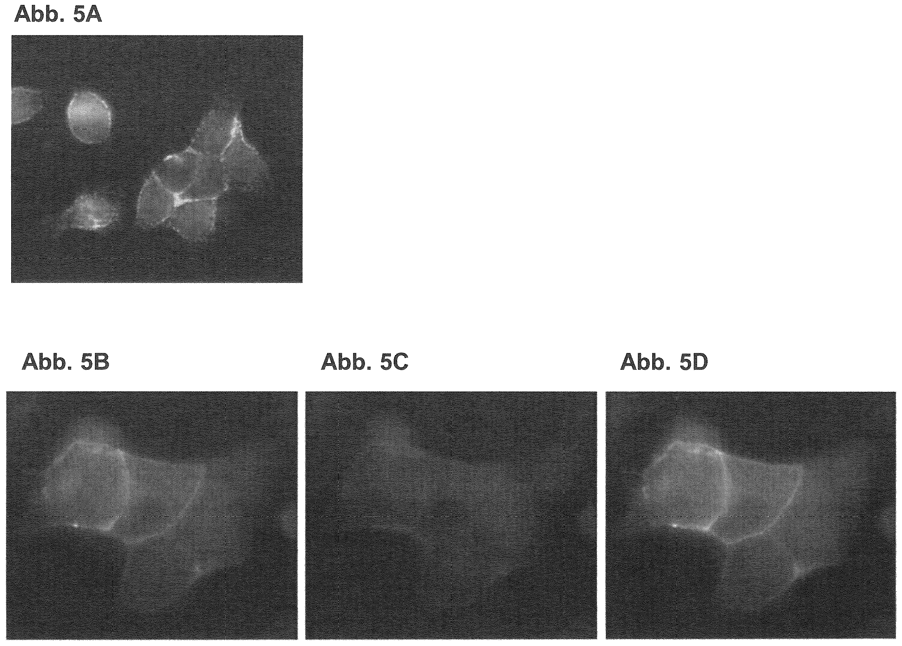

Beispielhaft werden die Daten für einen A2-spezifischen Antikörper, der durch Immunisierung mit SEQ ID NO: 17 hergestellt wurde, dargestellt. Der spezifische Antikörper lässt sich unter verschiedenen Fixierungsbedingungen für Immunfluoreszenz-Untersuchungen nutzen. Bei vergleichenden Färbungen von RT-PCR-positiven wie auch negativen-Zelllinien ist das entsprechende Protein in gut nachweisbarer Menge spezifisch u.a. in den als positiv typisierten Magentumor-, Ösophagustumor- und Pankreastumor-Zelllinien detektierbar (Abb. 5). Das endogene Protein ist membranlokalisiert und bildet größere fokale Aggregate an der Membran (Abb. 5). Mit diesem Antikörper wurden immunhistochemische Färbungen an menschlichen Geweben durchgeführt. Wir bestätigten die selektive Gewebeverteilung dieses Proteins. Ein umfangreiches Sortiment verschiedender Normalgewebe wurde untersucht, von denen in fast allen Claudin-18A2-Protein nicht nachweisbar ist, wie beispielhaft für Leber, Lunge, Niere und Kolon dargestellt wurde. Wir fanden lediglich eine Aktivierung dieses Proteins in normalem Magengewebe (Abb. 12). Überraschenderweise sahen wir, dass die A2-Variante von Claudin-18 zwar in den differenzierten Zellen der Magenschleimhaut nachweisbar ist, aber nicht in den Stammzellen. Differenzierte Magenschleimhautzellen sind einer ständigen Erneuerung unterworfen. Physiologischerweise wird das gesamte Magenepithel ausgehend von den Stammzellen des Magens kontinuierlich ersetzt. Dies unterstützt die Nutzbarkeit der A2-Variante als therapeutische Zielstuktur, da wir erfindungsgemäß zeigen, dass Stammzellen des Magens als die indispensible Zellpopulation der Magenschleimhaut genauso wie alle anderen gesunden Organe die A2-Variante nicht tragen und somit von einer spezifisch gegen die A2-Variante gerichteten Substanz nicht angegriffen werden können. Wir wiesen in einer Reihe humaner Tumoren mittels dieses Antikörpers die A2-Variante von Claudin-18 nach (Abb. 13), insbesondere in Tumoren von Magen, Ösophagus und Lunge, die uns bereits in RT-PCR-Untersuchungen aufgefallen waren. Erfindungsgemäß sind diese Tumoren therapeutisch zugänglich.

Der oben beschriebene Antikörper wurde des Weiteren für einen Proteinnachweis im Western-Blot eingesetzt. Erwartungsgemäß wird Protein lediglich in Magen und keinem anderen Normalgewebe, auch nicht Lunge, in der lediglich die A1-Variante aktiviert ist, detektiert (Abb. 9). Bei der vergleichenden Färbung von Magentumoren und adjazentem normalem Magengewebe aus Patienten fiel überraschend auf, dass in allen Magentumoren, in denen Claudin-18A2 detektiert wird, dieses Protein ein kleineres Massengewicht hat (Abb. 10, links). In einer Serie von Experimenten wurde erfindungsgemäß festgestellt, dass eine Bande auf dieser Höhe sich dadurch ergibt, dass man Lysat normalen Magengewebes mit dem deglykosylierenden Agens PNGase F behandelt (Abb. 10, rechts). Während in allen normalen Magengeweben ausschließlich die glykosylierte Form der Variante A2 nachweisbar ist, ist A2 als solches in über 60% der untersuchten Magentumore, und zwar ausschließlich in der deglykosylierten Form, nachweisbar. Obwohl die A2-Variante von Claudin-18 in normaler Lunge auch auf Proteinebene nicht detektiert wird, ist sie wie auch schon in der quantitativen RT-PCR in Lungentumoren zu finden. Auch hier liegt lediglich die deglykosylierte Variante vor (Abb. 11).

Claudin-18 selbst ist ein hochselektives Differenzierungsantigen von Magen (Variante A2) bzw. von Lunge und Magen (Variante A1). Unsere Daten weisen daraufhin, dass es offensichtlich von Tumor-assoziierten Veränderungen der Glykosylierungmaschinerie betroffen ist und in Tumoren eine besondere Form der Variante A2 entsteht, die deglykosyliert ist. Die Resultate der PNGaseF-Behandlung zeigen, dass Claudin-18A2 sich in Tumor-und Normalgewebe hinsichtlich seiner N-Glykosylierung unterscheidet.

Die Glykosylierung eines Epitops kann die Bindung eines für dieses Epitop-spezifischen Antikörpers verhindern und im vorliegenden Fall dazu beitragen, dass ein solcher Antikörper nicht an Claudin-18A2 in Normalgeweben binden kann, sondern exklusiv an die nicht-glykosylierte Form in Krebszellen. Um erfindungsgemäß Antikörper herzustellen, die exklusiv an nicht-glykosylierte Epitope binden, wurde dies bei der Auswahl der Immunogene berücksichtigt. Erfindungsgemäß haben wir verschiedene Regionen von Claudin-18A2 identifiziert, die in Tumor- und Normalgewebe unterschiedlich glykosyliert vorliegen können. Als potenzielle Glykosylierungsstellen für Claudin-18A2 haben wir u.a. die Regionen, die die Aminosäuren 37, 38, 45, 116, 141, 146, 205 von Claudin-18A2 umfassen, identifiziert (Abb. 2 unten). Erfindungsgemäß unterscheiden sich Tumorzellen und Normalgewebe durch eine unterschiedliche Glykosylierung in einer oder mehrerer dieser Positionen. Die meisten dieser Bereiche stellen keine klassische Glykosylierungsstelle dar, aber enthalten Asparagin, Serin und Threonin, die in seltenen Fällen auch glykosyliert werden können (Prädiktion Abb. 2 unten). Beide Varianten von Claudin-18 haben ein einziges klassisches Glykosylierungsmotiv in der D3-Domäne, die in der Literatur und entsprechend gängigen Prädiktionsalgorithmen als intrazellulär gilt. Allerdings konnte bei einem strukturell dem Claudin-18 ähnlichen Tetraspanin, dem PMP 22, gezeigt werden, dass die hydrophoben Membrandomänen 2 und 3 von PMP 22 nicht vollständig durch die Zellmembran laufen, sondern nur partiell in die Plasmamembran interkalieren (Taylor et al., J Neurosc. Res. 62:15-27, 2000). Aus diesem Grund ist der gesamte Bereich zwischen den zwei äusseren Transmembrandomänen bei PMP22 extrazellulär lokalisiert.

Wir haben die Möglichkeit einer solchen Topologie für Claudin-18A2 hypothetisiert und überprüft. Hierzu haben wir 3 Konstrukte hergestellt, die jeweils eine Marker-Sequenz (His- oder HA-Tag) in einer der Domänen EX1, EX2, oder D3 trugen (Abb. 15 oben). Diese wurden in Zelllinien transfiziert und daraufhin getestet, ob ein gegen diese Markersequenzen gerichteter Antikörper an nichtpermeabilisierte Zellen bindet, was erfordert, dass der entsprechende Bereich des Proteins topologisch extrazellulär ist. Da alle drei Bereiche des Moleküls durchflusszytometrisch als extrazellulär gemessen wurden (Abb. 15 unten), konnten wir bestätigen, dass Claudin-18A2 in einer Konformation mit zwei Transmembrandomänen und einer großen extrazellulär gelegenen Domäne vorliegen kann (Abb. 2, Konformation 2). Diese Konformation ist biochemisch und therapeutisch relevant, da sie zusätzliche Bindungsstellen für therapeutische Antikörper (SEQ ID NO: 142, 143) beinhaltet.

Erfindungsgemäß und vorzugsweise können Antikörper hergestellt werden, die zwischen glykosylierten und nicht glykosylierten Varianten von Claudin-18A2 unterscheiden. Diese haben eine besonders hohe Spezifität für Tumorzellen. Bei der Herstellung glykosilierungsspezifischer Antikörper haben wir neben den Glykosylierungsdomänen auch diese unterschiedlichen Konformationsoptionen berücksichtigt.

Vorzugsweise, aber nicht ausschließlich, eignen sich Proteinfragmente aus dem D3-Bereich von Claudin-18A2 zur Immunisierung von Tieren. Beispielhaft ist dies für zwei Antikörper mAB1 und mAB2 dargestellt (Abb. 17). Wir untersuchten die Bindeeigenschaften dieser Antikörper an Zelllinien, die entweder die A1- oder die A2-Variante des Claudin-18 exprimieren. Wir konnten zeigen, dass Claudin-18A2 für Antikörper auf der Zelloberfläche zugänglich ist. Erfindungsgemäss sind solche Antikörper spezifisch für die A2-Variante und binden nicht an die A1-Variante (Abb. 17). Wir haben kurze Fremdsequenzen (myc-tag) jeweils in den Bereich der Extrazellulärdomänen Ex1 und Ex2 eingeführt (Abb. 43). Am Beispiel von mAB1 ist dargestellt, dass die Bindeeigenschaften des Antikörpers dadurch nicht beeinträchtigt werden und das eigentliche Epitop sich in der D3-Domäne befindet. Die generierten Antikörper können diagnostisch wie auch therapeutisch genutzt werden. Immunseren wie das hier beschriebene (gegen Peptid SEQ ID NO: 17) können z.B. im Western-Blot diagnostisch genutzt werden. Erfindungsgemäß können durch Immunisierung mit Peptiden, die mindestens eine diese Regionen beinhalten (z.B. Peptid SEQ ID NO: 113 (Abbildung 6), Peptide SEQ ID NO: 142-145), Antikörper hergestellt werden, die an das glykosylierte Epitop gar nicht binden können. Erfindungsgemäß binden derartige Antikörper spezifisch an die deglykosylierten Epitope auf Tumorzellen. Die im Vergleich zu Normalgeweben fehlende Glykosylierung an einer der genannten Positionen könnte auch durch eine sekundäre, endogene Deglykosylierung in Tumorzellen bedingt sein. Eine derartige Deglykosylierung ist mit einer Asn (N)→ Asp (D) Transformation der betreffenden Aminosäure assoziiert. Für die Herstellung von Antikörpern gegen derartig veränderte tumorasssoziierte Varianten können daher erfindunggemäß Claudin-18A2-derivierte Peptide genutzt werden, in denen die Aminosäure Asn (N) an mindestens einer der Positionen 37, 38, 45, 116, 141, 146, 205 des Claudin-18A2-Peptids durch ein Asp (D) ersetzt ist (z.B. SEQ ID NO: 146-150). Insbesondere können solche Antikörper therapeutisch eingesetzt werden, da sie hochselektiv für Tumorzellen sind. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigenspezifischen T-Lymphozyten stellen die Aminosäuren 1-47 (SEQ ID NO: 19 und 120) besonders gute Epitope dar.

## Patentansprüche

1. Verwendung eines Antikörpers in einem Verfahren zur Detektion von Tumorzellen, die ein Tumor-assoziiertes Antigen exprimieren, wobei der Antikörper an das Tumor-assoziierte Antigen bindet,
wobei der Antikörper durch Immunisieren mit einem Peptid oder Polypeptid mit einer Aminosäuresequenz, die aus der Gruppe bestehend aus SEQ ID NOs: 142-148 ausgewählt ist, erhältlich ist, und wobei das im Tumorgewebe exprimierte Tumor-assoziierte Antigen die Aminosäuresequenz gemäß SEQ ID NO: 16 aufweist und im Vergleich zum gesunden Normalgewebe eine deglykosylierte Variante des Tumor-assoziierten Antigens darstellt, wobei die Glykosylierung eine N-Glykosylierung ist und der Tumor ein Magentumor oder ein Lungentumor ist und das Verfahren den Nachweis der deglykosylierten Variante des Tumor-assoziierten Antigens in einer biologischen Probe umfasst.

2. Verwendung nach Anspruch 1, wobei der Antikörper ein monoklonaler Antikörper ist.

3. Verwendung nach Anspruch 2, wobei der Antikörper ein chimärer oder humanisierter Antikörper oder ein Fragment eines Antikörpers ist, das selektiv an das Tumor-assoziierte Antigen bindet.

4. Verwendung nach einem der Ansprüche 1 bis 3, wobei der Antikörper mit einem diagnostischen Mittel gekoppelt ist.

## Claims

1. Use of an antibody in a method of detecting tumor cells expressing a tumor-associated antigen, wherein the antibody binds to the tumor-associated antigen,
wherein the antibody is obtainable by immunizing with a peptide or polypeptide having an amino acid sequence selected from the group consisting of SEQ ID NOs: 142-148, and wherein the tumor-associated antigen expressed in tumor tissue has the amino acid sequence according to SEQ ID NO: 16 and compared to healthy normal tissue is a deglycosylated variant of the tumor-associated antigen, wherein the glycosylation is a N-glycosylation and the tumor is a gastric tumor or a lung tumor, and the method comprises detecting the deglycosylated variant of the tumor-associated antigen in a biological sample.

2. The use of claim 1, wherein the antibody is a monoclonal antibody.

3. The use of claim 2, wherein the antibody is a chimeric or humanized antibody or a fragment of an antibody binding selectively to the tumor-associated antigen.

4. The use of any one of claims 1 to 3, wherein the antibody is coupled to a diagnostic agent.

## Revendications

1. Utilisation d'un anticorps dans un procédé pour la détection de cellules tumorales, qui expriment un antigène associé à une tumeur, tandis que l'anticorps se lie à l'antigène associé à une tumeur,
où l'anticorps peut être obtenu par immunisation avec un peptide ou polypeptide ayant une séquence d'acides aminés qui est choisie dans le groupe consistant en SEQ ID NOs: 142-148, et où l'antigène associé à une tumeur exprimé dans le tissu tumoral présente la séquence d'acides aminés selon SQ ID NO: 16 et par comparaison avec le tissu normal sain représente une variante déglycosylée de l'antigène associé à une tumeur, tandis que la glycosylation est une N-glycosylation et la tumeur est une tumeur gastrique ou une tumeur pulmonaire et le procédé comprend la détection de la variante déglycosylée de l'antigène associé à une tumeur dans un échantillon biologique.

2. Utilisation selon la revendication 1, où l'anticorps est un anticorps monoclonal.

3. Utilisation selon la revendication 2, où l'anticorps est un anticorps chimère ou humanisé ou un fragment d'un anticorps qui se lie sélectivement à l'antigène associé à une tumeur.

4. Utilisation selon l'une des revendications 1 à 3, où l'anticorps est couplé avec un agent de diagnostic.


### SEQUENZPROTOKOLL

<110> Ganymed Pharmaceuticals AG
<120> Differentiell in Tumoren exprimierte Genprodukte und deren Verwendung
<130> 342-23 EPT1
<150> DE 10 2004 024 617.3
   <151> 2004-05-18
<160> 32
<170> PatentIn version 3.1
<210> 7
   <211> 786
   <212> DNA
   <213> Homo sapiens
<400> 7
   atggccgtga ctgcctgtca gggcttgggg ttcgtggttt cactgattgg gattgcgggc      60
   atcattgctg ccacctgcat ggaccagtgg agcacccaag acttgtacaa caaccccgta     120
   acagctgttt tcaactacca ggggctgtgg cgctcctgtg tccgagagag ctctggcttc     180
   accgagtgcc ggggctactt caccctgctg gggctgccag ccatgctgca ggcagtgcga     240
   gccctgatga tcgtaggcat cgtcctgggt gccattggcc tcctggtatc catctttgcc     300
   ctgaaatgca tccgcattgg cagcatggag gactctgcca aagccaacat gacactgacc     360
   tccgggatca tgttcattgt ctcaggtctt tgtgcaattg ctggagtgtc tgtgtttgcc     420
   aacatgctgg tgactaactt ctggatgtcc acagctaaca tgtacaccgg catgggtggg     480
   atggtgcaga ctgttcagac caggtacaca tttggtgcgg ctctgttcgt gggctgggtc     540
   gctggaggcc tcacactaat tgggggtgtg atgatgtgca tcgcctgccg gggcctggca     600
   ccagaagaaa ccaactacaa agccgtttct tatcatgcct caggccacag tgttgcctac     660
   aagcctggag gcttcaaggc cagcactggc tttgggtcca acaccaaaaa caagaagata     720
   tacgatggag gtgcccgcac agaggacgag gtacaatctt atccttccaa gcacgactat     780
   gtgtaa                                                                786
<210> 8
   <211> 180
   <212> DNA
   <213> Homo sapiens
<400> 8
   tgcgccacca tggccgtgac tgcctgtcag ggcttggggt tcgtggtttc actgattggg      60
   attgcgggca tcattgctgc cacctgcatg gaccagtgga gcacccaaga cttgtacaac     120
   aaccccgtaa cagctgtttt caactaccag gggctgtggc gctcctgtgt ccgagagagc     180
<210> 16
   <211> 261
   <212> PRT
   <213> Homo sapiens
<400> 16
   Met Ala Val Thr Ala Cys Gln Gly Leu Gly Phe Val Val Ser Leu Ile
   1               5                   10                  15
   Gly Ile Ala Gly Ile Ile Ala Ala Thr Cys Met Asp Gln Trp Ser Thr
               20                  25                  30
   Gln Asp Leu Tyr Asn Asn Pro Val Thr Ala Val Phe Asn Tyr Gln Gly
           35                  40                  45
   Leu Trp Arg Ser Cys Val Arg Glu Ser Ser Gly Phe Thr Glu Cys Arg
       50                  55                  60
   Gly Tyr Phe Thr Leu Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 17
   <211> 10
   <212> PRT
   <213> Homo sapiens
<400> 17
   Asp Gln Trp Ser Thr Gln Asp Leu Tyr Asn
   1 5 10
<210> 18
   <211> 11
   <212> PRT
   <213> Homo sapiens
<400> 18
   Asn Asn Pro Val Thr Ala Val Phe Asn Tyr Gln
   1 5 10
<210> 19
   <211> 47
   <212> PRT
   <213> Homo sapiens
<400> 19
   Met Ala Val Thr Ala Cys Gln Gly Leu Gly Phe Val Val Ser Leu Ile
   1 5 10 15
   Gly Ile Ala Gly Ile Ile Ala Ala Thr Cys Met Asp Gln Trp Ser Thr
   20 25 30
   Gln Asp Leu Tyr Asn Asn Pro Val Thr Ala Val Phe Asn Tyr Gln
   35 40 45
<210> 39
   <211> 28
   <212> DNA
   <213> Künstliche Sequenz
<220>
   <223> Beschreibung der künstlichen Sequenz: Oligonukleotid
<400> 39
   ggttcgtggt ttcactgatt ggattgc 28
<210> 40
   <211> 27
   <212> DNA
   <213> Künstliche Sequenz
<220>
   <223> Beschreibung der künstlichen Sequenz: Oligonukleotid
<400> 40
   cggctttgta gttggtttct tctggtg 27
<210> 107
   <211> 20
   <212> DNA
   <213> künstliche Sequenz
<220>
   <223> Beschreibung der künstlichen Sequenz: Oligonukleotid
<400> 107
   tgttttcaac taccaggggc 20
<210> 108
   <211> 20
   <212> DNA
   <213> künstliche Sequenz
<220>
   <223> Beschreibung der künstlichen Sequenz: Oligonukleotid
<400> 108
   tgttggcttt ggcagagtcc 20
<210> 109
   <211> 24
   <212> DNA
   <213> künstliche Sequenz
<220>
   <223> Beschreibung der künstlichen Sequenz: Oligonukleotid
<400> 109
   gaggcagagt tcaggcttca ccga 24
<210> 110
   <211> 20
   <212> DNA
   <213> künstliche Sequenz
<220>
   <223> Beschreibung der künstlichen Sequenz: Oligonukleotid
<400> 110
   tgttggcttt ggcagagtcc 20
<210> 111
   <211> 56
   <212> PRT
   <213> Homo sapiens
<400> 111
   Thr Gly Met Asp Met Trp Ser Thr Gln Asp Leu Tyr Asp Asn Pro Val
   1               5                   10                  15
   Thr Ser Val Phe Gln Tyr Glu Gly Leu Trp Arg Ser Cys Val Arg Gln
               20                  25                  30
   Ser Ser Gly Phe Thr Glu Cys Arg Pro Tyr Phe Thr Ile Leu Gly Leu
           35                  40                  45
   Pro Ala Met Leu Gln Ala Val Arg
       50                  55
<210> 112
   <211> 53
   <212> PRT
   <213> Homo sapiens
<400> 112
   Asp Gln Trp Ser Thr Gln Asp Leu Tyr Asn Asn Pro Val Thr Ala Val
   1               5                   10                  15
   Phe Asn Tyr Gln Gly Leu Trp Arg Ser Cys Val Arg Glu Ser Ser Gly
               20                  25                  30
   Phe Thr Glu Cys Arg Gly Tyr Phe Thr Leu Leu Gly Leu Pro Ala Met
           35                  40                  45
   Leu Gln Ala Val Arg
       50
<210> 113
   <211> 14
   <212> PRT
   <213> Homo sapiens
<400> 113
   Ser Thr Gln Asp Leu Tyr Asn Asn Pro Val Thr Ala Val Phe
   1               5                   10
<210> 114
   <211> 12
   <212> PRT
   <213> Homo sapiens
<400> 114
   Asp Met Trp Ser Thr Gln Asp Leu Tyr Asp Asn Pro
   1 5 10
<210> 115
   <211> 12
   <212> PRT
   <213> Homo sapiens
<400> 115
   Cys Arg Pro Tyr Phe Thr Ile Leu Gly Leu Pro Ala
   1 5 10
<210> 116
   <211> 13
   <212> PRT
   <213> Homo sapiens
<400> 116
   Thr Asn Phe Trp Met Ser Thr Ala Asn Met Tyr Thr Gly
   1 5 10
<210> 117
   <211> 816
   <212> DNA
   <213> Homo sapiens
<400> 117
   gccaggatca tgtccaccac cacatgccaa gtggtggcgt tcctcctgtc catcctgggg 60
   ctggccggct gcatcgcggc caccgggatg gacatgtgga gcacccagga cctgtacgac 120
   aaccccgtca cctccgtgtt ccagtacgaa gggctctgga ggagctgcgt gaggcagagt 180
   tcaggcttca ccgaatgcag gccctatttc accatcctgg gacttccagc catgctgcag 240
   gcagtgcgag ccctgatgat cgtaggcatc gtcctgggtg ccattggcct cctggtatcc    300
   atctttgccc tgaaatgcat ccgcattggc agcatggagg actctgccaa agccaacatg    360
   acactgacct ccgggatcat gttcattgtc tcaggtcttt gtgcaattgc tggagtgtct    420
   gtgtttgcca acatgctggt gactaacttc tggatgtcca cagctaacat gtacaccggc    480
   atgggtggga tggtgcagac tgttcagacc aggtacacat ttggtgcggc tctgttcgtg    540
   ggctgggtcg ctggaggcct cacactaatt gggggtgtga tgatgtgcat cgcctgccgg    600
   ggcctggcac cagaagaaac caactacaaa gccgtttctt atcatgcctc aggccacagt    660
   gttgcctaca agcctggagg cttcaaggcc agcactggct ttgggtccaa caccaaaaac    720
   aagaagatat acgatggagg tgcccgcaca gaggacgagg tacaatctta tccttccaag    780
   cacgactatg tgtaatgctc taagacctct cagcac                              816
<210> 118
   <211> 261
   <212> PRT
   <213> Homo sapiens
<400> 118
   Met Ser Thr Thr Thr Cys Gln Val Val Ala Phe Leu Leu Ser Ile Leu
   1               5                   10                  15
   Gly Leu Ala Gly Cys Ile Ala Ala Thr Gly Met Asp Met Trp Ser Thr
               20                  25                  30
   Gln Asp Leu Tyr Asp Asn Pro Val Thr Ser Val Phe Gln Tyr Glu Gly
           35                  40                  45
   Leu Trp Arg Ser Cys Val Arg Gln Ser Ser Gly Phe Thr Glu Cys Arg
       50                  55                  60
   Pro Tyr Phe Thr Ile Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
                   85                  90                  95
   Ser Ile Phe Ala Leu Lys Cys Ile Arg Ile Gly Ser Met Glu Asp Ser
               100                 105                 110
   Ala Lys Ala Asn Met Thr Leu Thr Ser Gly Ile Met Phe Ile Val Ser
           115                 120                 125
   Gly Leu Cys Ala Ile Ala Gly Val Ser Val Phe Ala Asn Met Leu Val
       130                 135                 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 119
   <211> 227
   <212> DNA
   <213> Homo sapiens
<400> 119
   gccaggatca tgtccaccac cacatgccaa gtggtggcgt tcctcctgtc catcctgggg      60
   ctggccggct gcatcgcggc caccgggatg gacatgtgga gcacccagga cctgtacgac     120
   aaccccgtca cctccgtgtt ccagtacgaa gggctctgga ggagctgcgt gaggcagagt     180
   tcaggcttca ccgaatgcag gccctatttc accatcctgg gacttcc                   227
<210> 120
   <211> 69
   <212> PRT
   <213> Homo sapiens
<400> 120
   Met Ser Thr Thr Thr Cys Gln Val Val Ala Phe Leu Leu Ser Ile Leu
   1 5 10 15
   Gly Leu Ala Gly Cys Ile Ala Ala Thr Gly Met Asp Met Trp Ser Thr
   20 25 30
   Gln Asp Leu Tyr Asp Asn Pro Val Thr Ser Val Phe Gln Tyr Glu Gly
   35 40 45
   Leu Trp Arg Ser Cys Val Arg Gln Ser Ser Gly Phe Thr Glu Cys Arg
   50 55 60
   Pro Tyr Phe Thr Ile
   65
<210> 137
   <211> 32
   <212> PRT
   <213> Homo sapiens
<400> 137
   Asn Met Leu Val Thr Asn Phe Trp Met Ser Thr Ala Asn Met Tyr Thr
   1 5 10 15
   Gly Met Gly Gly Met Val Gln Thr Val Gln Thr Arg Tyr Thr Phe Gly
   20 25 30
<210> 142
   <211> 116
   <212> PRT
   <213> Homo sapiens
<400> 142
   Ala Leu Met Ile Val Gly Ile Val Leu Gly Ala Ile Gly Leu Leu Val
   1               5                   10                  15
   Ser Ile Phe Ala Leu Lys Cys Ile Arg Ile Gly Ser Met Glu Asp Ser
               20                  25                  30
   Ala Lys Ala Asn Met Thr Leu Thr Ser Gly Ile Met Phe Ile Val Ser
           35                  40                  45
   Gly Leu Cys Ala Ile Ala Gly Val Ser Val Phe Ala Asn Met Leu Val
       50                  55                  60
   Thr Asn Phe Trp Met Ser Thr Ala Asn Met Tyr Thr Gly Met Gly Gly
   65                  70                  75                      80
   Met Val Gln Thr Val Gln Thr Arg Tyr Thr Phe Gly Ala Ala Leu Phe
                   85                  90                  95
   Val Gly Trp Val Ala Gly Gly Leu Thr Leu Ile Gly Gly Val Met Met
               100                 105                 110
   Cys Ile Ala Cys
           115
<210> 143
   <211> 24
   <212> PRT
   <213> Homo sapiens
<400> 143
   Arg Ile Gly Ser Met Glu Asp Ser Ala Lys Ala Asn Met Thr Leu Thr
   1               5                   10                  15
   Ser Gly Ile Met Phe Ile Val Ser
               20
<210> 144
   <211> 8
   <212> PRT
   <213> Homo sapiens
<400> 144
   Ala Lys Ala Asn Met Thr Leu Thr
   1               5
<210> 145
   <211> 14
   <212> PRT
   <213> Homo sapiens
<400> 145
   Met Glu Asp Ser Ala Lys Ala Asn Met Thr Leu Thr Ser Gly
   1               5                   10
<210> 146
   <211> 14
   <212> PRT
   <213> Homo sapiens
<400> 146
   Met Glu Asp Ser Ala Lys Ala Asp Met Thr Leu Thr Ser Gly
   1               5                   10
<210> 147
   <211> 9
   <212> PRT
   <213> Homo sapiens
<400> 147
   Ser Ala Lys Ala Asp Met Thr Leu Thr
   1               5
<210> 148
   <211> 9
   <212> PRT
   <213> Homo sapiens
<400> 148
   Ala Lys Ala Asp Met Thr Leu Thr Leu
   1               5
<210> 149
   <211> 53
   <212> PRT
   <213> Homo sapiens
<400> 149
   Asp Gln Trp Ser Thr Gln Asp Leu Tyr Asp Asn Pro Val Thr Ala Val
   1               5                   10                  15
   Phe Asn Tyr Gln Gly Leu Trp Arg Ser Cys Val Arg Glu Ser Ser Gly
               20                  25                  30
   Phe Thr Glu Cys Arg Gly Tyr Phe Thr Leu Leu Gly Leu Pro Ala Met
           35                  40                  45
   Leu Gln Ala Val Arg
       50
<210> 150
   <211> 14
   <212> PRT
   <213> Homo sapiens
<400> 150